# EUROPEAN PATENT SPECIFICATION

(11) **EP 4 410 215 B1**
(45) Date of publication and mention of the grant of the patent: **08.04.2026**
(21) Application number: 24155568.9
(22) Date of filing: 02.02.2024
(51) Int. Cl.: A61B 8/02, A61B 5/0245, A61B 8/06, A61B 8/08, A61B 8/00

(54) **NON-VISUAL ALERTS AND INDICATORS OF MEDICAL CONDITIONS**
NICHTVISUELLE ALARME UND INDIKATOREN FÜR MEDIZINISCHE ZUSTÄNDE
ALERTES ET INDICATEURS NON VISUELS D'ÉTATS MÉDICAUX

(30) Priority: 03.02.2023 US 202363443301 P; 28.12.2023 US 202363615730 P
(43) Date of publication of application: 07.08.2024
(73) Proprietor: Stryker Corporation, Portage, MI 49002-9711 (US)
(72) Inventor: SIEDENBURG, Clinton T., Kalamazoo, 49002 (US); CHAPMAN, Fred W., Kalamazoo, 49002 (US); PIRAINO, Daniel W., Kalamazoo, 49002 (US); TAYLOR, Tyson G., Kalamazoo, 49002 (US)
(74) Representative: V.O.

(56) References cited:
- US-A1- 2011 245 651
- US-A1- 2018 153 514
- US-A1- 2021 059 638

## Description

### TECHNICAL FIELD

The present application relates to a medical device, comprising:
a first sensor configured to detect, during a time interval, a sound indicative of cardiac activity of a subject;
a second sensor configured to detect, during the time interval, an additional physiological parameter of the subject;
a processor configured to generate audio indicative of a pulse of the subject by analyzing the sound indicative of cardiac activity of the subject;
a speaker configured to output the audio indicative of the pulse of the subject; and
a display configured to output a visual signal indicating the additional physiological parameter of the subject detected during the time interval, the display outputting the visual signal simultaneously as the speaker is outputting the audio indicative of the pulse of the subject.

The present application also relates to a method performed by such a medical device.

### BACKGROUND

Such a medical device is described in US 2011/245651 that discloses obtaining time-series images of a heart and sound data representing the heart sounds. The time-series images and sound data representing the heart are synchronized with each other and are played back on a display and on a speaker.

Medical devices are often configured to convey information to users. For example, monitor-defibrillators are configured to display multiple physiological parameters of a patient, in real-time, on a screen. The multiple physiological parameters, in some cases, enable a user to identify complex medical conditions of the patient. For instance, the presence of QRS complexes in an electrocardiogram (ECG) indicate either pulseless electrical activity (PEA) or a return of spontaneous circulation (ROSC). A rescuer, for instance, is able to distinguish whether a patient has PEA or ROSC by also reviewing a blood pressure of the patient along with the ECG.

### SUMMARY

According to an aspect is provided a medical device according to claim 1.

Optionally, the sound indicative of cardiac activity of the subject comprises a reflection of an incident beam from a heart of the subject.

Optionally, the first sensor comprises an ultrasound transducer and the incident beam comprises ultrasound.

Optionally, the processor is configured to generate the audio indicative of the pulse of the subject by determining a velocity of a heart wall of the subject by comparing a frequency of the reflection of the incident beam to a frequency of the incident beam. The processor is configured to generate the audio indicative of the pulse of the subject by determining a velocity of a heart wall of the subject by comparing a frequency of the reflection of the incident beam to a frequency of the incident beam, and defining a pitch or volume of the audio to be proportional to the velocity of the heart wall of the subject.

Optionally, the reflection of the incident beam is from: blood flowing through a blood vessel of the subject; blood flowing through a heart of the subject; and/or a wall of the heart of the subject.

Optionally, the physiological parameter comprises an electrocardiogram (ECG), a blood oxygenation, or a capnograph.

Optionally, the audio indicative of the pulse of the subject is further indicative of an arrhythmia of a heart of the subject during the time interval.

Optionaly, the processor is configured to generate the audio indicative of the pulse of the subject by analyzing the sound indicative of cardiac activity of the subject by determining a difference between a rate of the pulse and a reference value, and generating the audio to have a pitch that is proportional to the difference.

Optionally, the medical device further comprises a monitoring circuit configured to detect an electrocardiogram (ECG) of a subject, and an input device configured to receive an input signal from a user. The display can be configured to visually output the ECG and the physiological parameter. The processor can be configured to determine that the ECG is indicative of an arrhythmia. The processor can be configured to, in response to determining that the ECG is indicative of an arrhythmia and the input device receiving the input signal from the user, generate a command to output an electrical shock to the heart of the subject. The medical device can further comprise a treatment circuit configured to, in response to the processor determining that the ECG is indicative of an arrhythmia and the input device receiving the input signal from the user, output an electrical shock to the heart of the subject.

Optionally, the first sensor comprises a transmitter configured to output ultrasound toward the heart of the subject and a receiver configured to detect a reflection of the ultrasound from blood in the heart of the subject, the sound being the reflection of the ultrasound from the blood in the heart of the subject. Optionally, the medical device further comprises a housing and an adhesive disposed on the housing. The adhesive can be configured for attaching the first sensor to skin, e.g. on a chest, of the subject. The processor can be further configured to determine a velocity of the blood in the heart of the subject over time by analyzing the sound. The processor can be configured to convert the velocity of the blood in the heart of the subject over time into the audio.

According to an aspect is provided a monitor-defibrillator, comprising a monitoring circuit configured to detect an electrocardiogram (ECG) of a subject; a parameter sensor configured to detect a physiological parameter of the subject; a display configured to visually output the ECG and the physiological parameter; a sound sensor configured to detect a sound from a heart of a subject; a speaker configured to output audio indicative of the sound from the heart of the subject; an input device configured to receive an input signal from a user; a processor configured to determine that the ECG is indicative of an arrhythmia; and a treatment circuit configured to, in response to the processor determining that the ECG is indicative of an arrhythmia and the input device receiving the input signal from the user, output an electrical shock to the heart of the subject.

Optionally, the sound sensor comprises a transmitter configured to output ultrasound toward the heart of the subject; a receiver configured to detect a reflection of the ultrasound from blood in the heart of the subject, the sound being the reflection of the ultrasound from the blood in the heart of the subject; a housing; and an adhesive disposed on the housing. The adhesive can be configured fot attaching the sound sensor to skin, e.g. on a chest, of the subject. The processor is further configured to: determine a velocity of the blood in the heart of the subject over time by analyzing the sound; and convert the velocity of the blood in the heart of the subject over time into the audio.

Optionally, the audio is indicative of the arrhythmia.

According to an aspect is provided a method according to claim 10.

Optionally, the sound indicative of cardiac activity of the subject comprises a reflection of an incident beam from a heart of the subject. The incident beam can comprise ultrasound. Generating the audio indicative of the pulse of the subject can comprise determining a velocity of a wall of a heart of the subject by comparing a frequency of the reflection of the incident beam to a frequency of the incident beam, and defining a pitch or volume of the audio to be proportional to the velocity of the wall of the heart of the subject. The reflection of the incident beam can be from blood flowing through a heart of the subject or a blood vessel of the subject.

Optionally, the sound indicative of cardiac activity of the subject is generated by movement of a heart of the subject and/or by blood flowing through a blood vessel of the subject.

Optionally, the additional physiological parameter comprises an electrocardiogram
(ECG), a blood oxygenation, or a capnograph.

Optionally, the audio indicative of the pulse of the subject is further indicative of an arrhythmia of a heart of the subject during the time interval

Generating the audio indicative of the pulse of the subject by analyzing the sound indicative of cardiac activity of the subject comprises determining a difference between a rate of the pulse and a reference value, and defining a pitch or volume of the audio to be proportional to the difference.

It will be appreciated that any of the aspects, features and options described herein can be combined. It will particularly be appreciated that any of the aspects, features and options described in view of the medical device apply equally to the monitor defibrillator and method, and vice versa.

### BRIEF DESCRIPTION OF THE DRAWINGS

FIG. 1 illustrates an example environment for conveying patient-relevant information using audio.
FIG. 2 illustrates an example environment for monitoring blood flow through one or more blood vessels of a subject.
FIG. 3 illustrates various placements of a flow monitor on the body of a patient.
FIG. 4 illustrates the structure of an example flow monitor.
FIG. 5 illustrates an example process for conveying physiological parameters using both audio and a visual signal.
FIG. 6 illustrates an example process for conveying a condition of a subject based on a detected sound.
FIG. 7 illustrates an example process for indicating a severity of a condition of a subject by outputting an audio signal.
FIG. 8 illustrates an example of an external defibrillator configured to perform various functions described herein.
FIG. 9 illustrates a chest compression device configured to perform various functions described herein
FIG. 10 illustrates a flow monitor configured to perform various functions described herein.

### DETAILED DESCRIPTION

Various implementations described herein relate to techniques for communicating patient conditions in a non-visual manner. Existing medical devices convey physiological parameters and other patient information visually, such as on a display. In emergency settings and other high-stress environments, it may be difficult for rescuers to analyze multiple physiological parameters of a patient displayed on a screen in real-time. In some cases, a rescuer may miss an indicator of a deteriorating condition of the patient due to being distracted by a significant amount of physiological parameters displayed on a single screen of a medical device.

In implementations of the present disclosure, at least some information indicative of physiological parameters and/or conditions of a monitored patient is conveyed to a user using audio signals. In some examples, a medical device outputs an audio signal indicative of a pulse of a subject while also displaying an additional physiological parameter of the subject. In some cases, a medical device selectively outputs a sound detected at a portion of a body of a subject, in response to determining that the sound is indicative of a condition of the subject. For instance, implementations of the present disclosure can be utilized as a "smart stethoscope" that selectively outputs audio from the subject with diagnostic relevance, and that refrains from outputting audio that has limited diagnostic relevance. In some examples, a medical device outputs an audio signal having a characteristic that is proportional to a variance between a detected physiological parameter and a reference value. In various instances described herein, medical devices may alert rescuers to changes in patient conditions efficiently using audio signals.

Implementations of the present disclosure are directed to improvements in the technical field of patient monitoring, particularly in emergency medical settings. In previous technologies, medical devices utilized in emergency settings (e.g., monitor-defibrillators) would display physiological parameters and related alerts visually. Although these devices occasionally would output audio signals, such as alerts, the audio signals did not enable a listening rescuer to directly diagnose the extent of a condition a monitored patient. For example, a previous audio alert could include a synthetic, high-pitched beep when the device detected a heart rate of the patent to be over a threshold, but such an alert could not be analyzed by the rescuer to determine whether the patient had VF (which could be detected as a high heart rate) or tachyarrhythmia. Furthermore, the alert could not convey, to the rescuer, a severity of the patient's condition, such as a magnitude of the high heart rate. Thus, previous technologies would rely on the rescuer visually observing physiological parameters of the patient on a display, for instance, in order to identify a condition of the patient. According to various implementations herein, a medical device can output an audio alert that directly conveys patient information, such as a magnitude or type of physiological parameter detected by the medical device, thereby enabling the identification and diagnosis of conditions that were not previously conveyed with sound. Accordingly, implementations of the present disclosure can enable a single rescuer to identify the condition of the patient without visually reviewing detected physiological parameters, particularly in emergency settings.

As used herein, the terms "blood flow," "blood flow parameters," and their equivalents, may refer to one or more physiological parameters indicative of a movement of blood through a blood vessel. For example, the term "blood velocity" may refer a change in position with respect to time (i.e., distance per time, such as meters per second) of one or more blood cells moving in a blood vessel. The term "blood speed" may refer to a magnitude of a velocity (i.e., distance per time) of one or more blood cells moving in a blood vessel. The term "velocity profile," for example, may refer to a velocity of blood in a blood vessel with respect to a location within the blood vessel, such as a velocity of blood in a blood vessel with respect to a location along a cross-section of the blood vessel. The term "flow rate," and its equivalents, may refer to a volume or mass of blood that passes a boundary (e.g., a cross-section of a blood vessel) with respect to time. The terms "net flow," "net flow volume," and their equivalents, may refer to a volume or mass of blood that passes a boundary during a discrete time interval, such as during a cardiac cycle. A net flow volume can be calculated by integrating a flow rate over the time interval. Unless otherwise specified explicitly or by context, the term "blood flow" may refer to blood velocity, blood speed, velocity profile, flow rate, net flow, or any other flow-related parameter described herein.

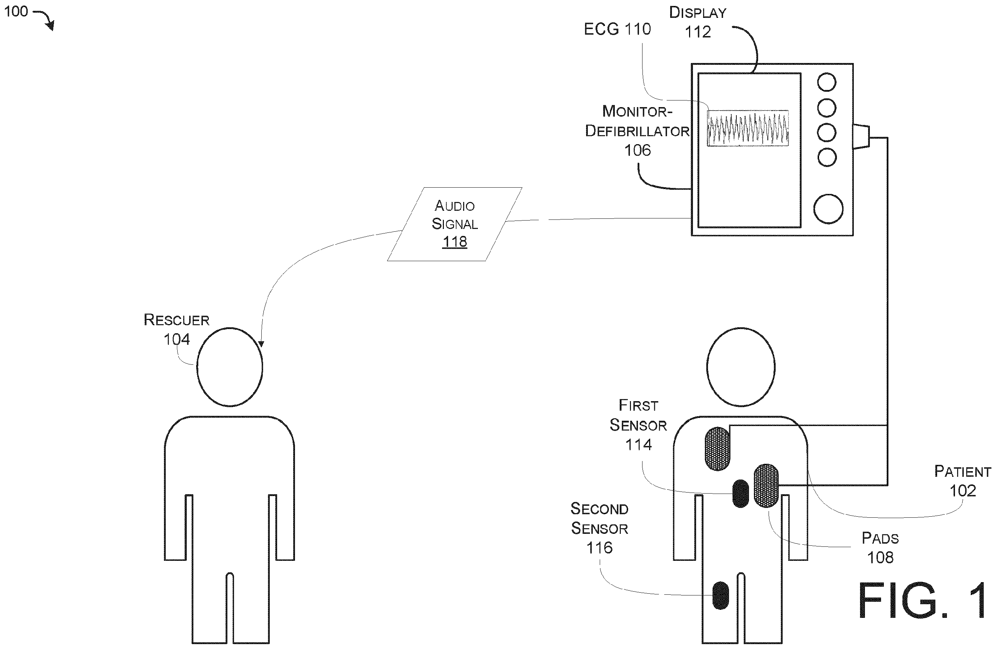

FIG. 1 illustrates an example environment 100 for conveying patient-relevant information using audio. The environment 100 includes a patient 102 being monitored and/or treated by a rescuer 104. In various implementations, the environment 100 is a nonclinical environment. For example, the patient 102 may be experiencing an unexpected medical emergency in the environment 100, such that the rescuer 104 was deployed to the environment 100 via an emergency services system. In some examples, the rescuer 104 is a trained emergency medical technician (EMT), clinician, or other type of caregiver. For instance, the rescuer 104 may have arrived at the environment 100 in an ambulance or other emergency services transporter.

Specifically, in the example of FIG. 1, the rescuer 104 operates a monitor-defibrillator 106 in order to monitor the patient 102. In various cases, the monitor-defibrillator 106 is configured to identify a condition of the patient 102. For example, the monitor-defibrillator 106 may be configured to determine if the patient 102 is experiencing a cardiac arrhythmia or other pathology. In some examples, the monitor-defibrillator 106 is further configured to administer a therapy to the patient 102. For instance, the monitor-defibrillator 106 may be configured to output one or more electrical shocks to the heart of the patient 102 as part of a pacing, defibrillation, or synchronized cardioversion therapy. In various cases, the monitor-defibrillator 106 is an external defibrillator. The monitor-defibrillator 106, in some examples, includes an on-board power supply (e.g., one or more batteries) and is portable. For instance, the rescuer 104 may carry the monitor-defibrillator 106 into the environment 100 for use with monitoring and treating the patient 102.

The monitor-defibrillator 106 is connected to pads 108 that are adhered to the chest of the patient 102. In particular cases, the pads 108 are disposed on the skin of the patient 102. The pads 108, for instance, include multiple electrodes that are disposed at positions on the chest of the patient 102 that enable the electrodes to detect an electrical signal from the heart of the patient 102, and that enable the electrodes to deliver an electrical signal (e.g., an electrical shock) to the heart of the patient 102.

Using the pads 108, the monitor-defibrillator 106 detects an electrical signal from the heart of the patient 102 that is indicative of an electrocardiogram (ECG) 110 of the patient 102. The ECG 110 may include one or more leads. The ECG 110, for example, is a 12-lead or 15-lead ECG. As used herein, the term "lead," and its equivalents, may refer to a relative electrical potential over time between at least two electrodes positioned at predetermined positions along the body of an individual, such that the relative electrical potential is indicative of the electrical activity of the individual's heart. For example, the pads 108 are positioned such that the electrodes are disposed at predetermined ECG lead positions. The pads 108 may be disposed at locations corresponding to a bipolar limb lead (e.g., Lead I, Lead II, Lead III), a unipolar limb lead (e.g., Lead aVR, Lead aVL, Lead aVF), a unipolar chest lead (e.g., Lead V1, Lead V2, Lead V3, Lead V4, Lead V5, Lead V6). In various cases, the monitor-defibrillator 106 generates data indicative of the ECG 110 based on the detected electrical signal from the heart of the patient 102.

The monitor-defibrillator 106 visually presents the ECG 110 on a display 112. In various cases, the display 112 includes a screen configured to visually present the ECG 110 and other information to the rescuer 104. For instance, the monitor-defibrillator 106 presents the ECG 110 as a continuous waveform on the display 112. In some examples, the display 112 presents a portion of the ECG 110 detected within a particular time period, such as 5 seconds, 10 seconds, 30 seconds, one minute, or the like. In some cases, the monitor-defibrillator 106 includes one or more input devices enabling the rescuer 104 to adjust the portion of the ECG 110 that is output on the display 112. For instance, the display 112 may be a touchscreen including touch sensors configured to receive an input signal (e.g., a touch signal) from the rescuer 104, and the monitor-defibrillator 106 may adjust the amount of the ECG 110 that is output on the display 112 based on the input signal.

The ECG 110 is an example of a physiological parameter of the patient 102 that is detected by the monitor-defibrillator 106. As used herein, the term "physiological parameter," and its equivalents, may refer to a metric indicative of a characteristic of the body or health of an individual. Other examples of physiological parameters include a sound (e.g., an audible sound or ultrasound) emitted by at least a portion of the individual, a movement (e.g., an acceleration) of at least a portion of the individual, a blood pressure (e.g., non-invasive or invasive blood pressure), a blood flow (e.g., blood velocity) through at least one blood vessel of the individual, an airway parameter (e.g., a respiration rate, a capnograph, an end-tidal carbon dioxide, an end-tidal oxygen, a partial pressure of carbon dioxide, or a partial pressure of oxygen), a plethysmograph, a blood oxygenation (e.g., a regional oxygenation or a pulse oxygenation), a heart rate, a pulse rate, a temperature (e.g., a core temperature), or any combination thereof.

Additional sensors detect one or more additional physiological parameters of the patient 102. A first sensor 114 detects a physiological parameter of the patient 102 at a first location on the body of the patient 102. A second sensor 116, for instance, detects the physiological parameter of the patient 102 at a second location on the body of the patient 102. In various cases, the first sensor 114 is disposed on and/or in the body of the patient 102 at a position that is adjacent to the first location. The second sensor 116, for instance, is disposed on and/or in the body of the patient 102 at a position that is adjacent to the second location. Various types of devices may be utilized as the first sensor 114 and the second sensor 116. For example, the first sensor 114 and/or the second sensor 116 may include at least one of a microphone, a transducer (e.g., an ultrasound transducer), an infrared sensor, a blood pressure sensor (e.g., a blood pressure cuff or ultrasound-based blood pressure sensor), a blood oxygenation sensor (e.g., at least one photosensor configured to detect light transmitted through and/or scattered from a portion of the body of the patient 102 from at least one light source, the transmitted and/or scattered light being indicative of an amount of oxygen bound to hemoglobin in the blood of the patient 102), a flow sensor (e.g., an airway flow rate and/or velocity), an accelerometer (e.g., a pulse sensor), one or more electrodes, a gas sensor (e.g., a carbon dioxide or oxygen sensor), or any combination thereof. The first location and the second location, in various cases, may include a heart, a lung, an airway, an abdomen, a chest, a limb, a neck, an organ, a head, or any combination thereof. In some cases, the first sensor 114 and the second sensor 116 are the same type of sensor.

The first sensor 114 and the second sensor 116, in various cases, sample the physiological parameter periodically, repeatedly, and/or in response to events. For example, the first sensor 114 and the second sensor 116 may measure the physiological parameter at a predetermined sampling frequency. According to some cases, the first sensor 114 and the second sensor 116 sample the physiological parameter continuously.

The first sensor 114 and the second sensor 116 are communicatively coupled with the monitor-defibrillator 106. In some examples, the first sensor 114 and the second sensor 116 include respective transceivers configured to transmit, to the monitor-defibrillator 106, communication signals indicative of the sampled physiological parameter. In some cases, the first sensor 114 and the second sensor 116 stream data indicating the sampled physiological parameter to the monitor-defibrillator 106 substantially in real-time. For example, the monitor-defibrillator 106 may receive data indicating a measurement of the physiological parameter within 0.1 seconds of the measurement being detected by the first sensor 114 or the second sensor 116. In some cases, the first sensor 114 and/or the second sensor 116 is connected to the monitor-defibrillator 106 via a wired connection, such as an electrical or optical connection. Thus, the first sensor 114 and the second sensor 116 may communicate the detected physiological parameter with the monitor-defibrillator 106 in a wireless fashion and/or a wired fashion.

In some cases, the monitor-defibrillator 106 visually presents the physiological parameter detected by the first sensor 114 and/or the second sensor 116 on the display 112. For instance, the display 112 may visually present one or more waveforms, alerts, numerals, or the like that are indicative of the sampled physiological parameter.

According to some cases, the patient 102 is experiencing an acute medical emergency. For example, the patient 102 may be exhibiting episodes of VF. The rescuer 104 may need to constantly view the ECG 110 in order to manage the condition of the patient 102 in the medical emergency. For example, the rescuer 104 may have to actively review the ECG 110 for the VF episodes and administer an electrical shock to the patient 102 using the monitor-defibrillator 106 in response to the VF episodes. In this medical emergency, the rescuer 104 may be unable to view other waveforms or user interface elements visually presented on the display 112. As a result, any display of the physiological parameter detected by the first sensor 114 or the second sensor 116 may be ignored or even impossible to review by the rescuer 104.

In various implementations of the present disclosure, rather than (e.g., exclusively) displaying the physiological parameter on the display 112, the monitor-defibrillator 106 outputs an audio signal 118 to the rescuer 104. The audio signal 118, in various cases, may reflect the physiological parameter detected by the first sensor 114 and/or the second sensor 116. According to various implementations, the audio signal 118 enables the rescuer 104 to elucidate a complex condition of the patient 102 indicated by the physiological parameter detected by the first sensor 114 and/or the second sensor 116, even when the rescuer 104 is visually focused on the ECG 110 displayed on the display 112, on the patient 102, or on some other element capturing the visual attention of the rescuer 104.

The monitor-defibrillator 106 may generate the audio signal 118 based on the physiological parameter. In some implementations, the physiological parameter is indicative of cardiac activity of the patient 102. For instance, the physiological parameter includes a sound corresponding to a movement of blood through at least one blood vessel of the patient 102, motion of a heart wall of the patient 102, motion of blood through the heart of the patient 102, or any combination thereof. The sound, for example, is a sound emitted from the blood, the blood vessel, or heart of the patient 102.

In some examples, the detected sound is based on a reflection from the blood, the blood vessel, or heart of the patient 102. For instance, the first sensor 114 or the second sensor 116 emits an incident beam toward the blood, the blood vessel, or heart of the patient 102. The incident beam, for instance, includes ultrasound and/or light. The first sensor 114 or the second sensor 116, in various cases, detects a reflection of the incident beam from the blood, the blood vessel, or the heart of the patient 102. Based on the detected reflection of the incident beam, the first sensor 114 or the second sensor 116 may detect movement of the blood, the blood vessel, or the heart of the patient 102. For example, the first sensor 114 or the second sensor 116 may determine a difference between the frequency and/or phase of the incident beam to the frequency and/or phase of the reflection, and may determine a magnitude of the movement (e.g., a velocity of the blood, a wall of the blood vessel, or a wall of the heart) based on the difference. In some examples, the first sensor 114 is configured to detect movement from a first portion of the patient 102 and the second sensor 116 is configured to detect movement from a second portion of the patient 102.

According to some cases, the audio signal 118 is generated based on the physiological parameter. The monitor-defibrillator 106, in various implementations, generates the audio signal 118 to have a characteristic that is dependent on a magnitude of the physiological parameter. For instance, a pitch, volume, quality, or other characteristic of the audio signal 118 may be proportional or otherwise dependent on the magnitude of the physiological parameter. In some examples, the audio signal 118 includes two sounds, such that a harmony or desynchrony between the two sounds is proportional or otherwise dependent on the magnitude of the physiological parameter. In some examples, the magnitude of the physiological parameter changes over time. For instance, the velocity of blood through a blood vessel of the patient 102 may change over the course of a cardiac cycle of the patient 102. According to various cases, a characteristic (e.g., pitch) of the audio signal 118 also changes accordingly over the course of the cardiac cycle. In some cases, the monitor-defibrillator 106 detects blood flow or heart sounds from successive cardiac cycles. The monitor-defibrillator 106, in some cases, generates the audio signal 118 by comparing the sounds from the successive cardiac cycles. For example, the audio signal 118 is indicative of a difference in frequencies of the sounds of the successive cardiac cycles.

The first sensor 114 and the second sensor 116 may be detecting the physiological parameter simultaneously. In some examples, the audio signal 118 output at a particular time is indicative of the physiological parameter detected by the first sensor 114 or the second sensor 116. The monitor-defibrillator 106, in various instances, is configured to select, weigh, or prioritize the physiological parameter detected by the first sensor 114 or the physiological parameter detected by the second sensor 116 at the same time. For example, the monitor-defibrillator 106 analyzes first data indicative of the physiological parameter detected by the first sensor 114 and second data indicative of the physiological parameter detected by the second sensor 116. In various cases, the monitor-defibrillator 106 generates the audio signal 118 based on the physiological parameter detected by the first sensor 114 if the monitor-defibrillator 106 determines that the first data is indicative of a condition. Examples of conditions include a cardiac arrhythmia (e.g., VF, VT, bradycardia, tachycardia, atrial fibrillation (AF), etc.), an abnormal breath sound (e.g., rales, rhonchi, stridor, wheezing, absence of breath sounds, etc.), an airway obstruction, a myocardial infarction, absence of a pulse, internal bleeding, or any combination thereof.

In some cases, the monitor-defibrillator 106 is configured to determine that data is indicative of a condition by determining and analyzing a characteristic of the detected physiological parameter. In some implementations, the characteristic includes a magnitude, a frequency, a spectral component, or any combination thereof, of the detected physiological parameter. For example, the monitor-defibrillator 106 may determine that the data is indicative of a condition if at least one measurement of the physiological parameter is above a first threshold or below a second threshold. In some cases, the monitor-defibrillator 106 determines that the data is indicative of a condition if a frequency of the data is above a third threshold or below a fourth threshold. According to some cases, the monitor-defibrillator 106 performs a transform (e.g., a wavelet transform, a Fourier transform, a Laplace transform, or the like) on the data, and determines that the condition is present if a spectral component of the transformed data is above a fifth threshold or below a sixth threshold.

If both the first data and the second data are indicative of conditions, the monitor-defibrillator 106 may determine which condition is more important for the rescuer 104 to consider, and may generate the audio signal 118 based on the more important data. In some cases, the first data and the second data are indicative of the same condition, but the more important data may be the data indicating a greater magnitude of the condition. In some implementations, the condition indicated by the first data is different than the condition indicated by the second data. For instance, the monitor-defibrillator 106 may store a look-up table listing different conditions in order of priority, and the more important data may correspond to the data indicating a prioritized condition over the other data indicating the other condition. In other words, the monitor-defibrillator 106 may generate the audio signal 118 based on data among the first data and the second data with the greatest priority.

The conditions indicated by the physiological parameter, and their priorities, may change over time. In various implementations, the monitor-defibrillator 106 may constantly or periodically reevaluate which of the first data and the second data has the higher priority, and adjust the audio signal 118 accordingly. For example, at a first time, the first data may indicate a condition and the second data may not indicate a condition. However, at a second time, the second data may indicate the condition and the first data may not indicate the condition. Accordingly, the monitor-defibrillator 106 may generate the audio signal 118 based on the first data detected at the first time, and may later generate the audio signal 118 based on the second data detected at the second time. Thus, the monitor-defibrillator 106 may selectively generate the audio signal 118 based on real-time conditions of the patient 102.

Particular implementations will now be described with respect to FIG. 1. For instance, the rescuer 104 presents to the environment 100 in response to an emergency call reporting that the patient 102 has suddenly collapsed in an airport terminal. The rescuer 104 physically carries the monitor-defibrillator 106, the first sensor 114, and the second sensor 116 to the environment. In various cases, each one of the monitor-defibrillator 106, the first sensor 114, and the second sensor 116 is portable and may be powered via one or more batteries. The rescuer 104 applies the pads 108 to the chest of the patient 102. The rescuer 104 further applies the first sensor 114 to the chest of the patient 102 and the second sensor 116 to a leg of the patient 102. For instance, the rescuer 104 unpeels covers from adhesive layers disposed on housings of of the first sensor 114 and second sensor 116, and places the first sensor 114 and the second sensor 116 on the skin of the patient 102, thereby causing the first sensor 114 and the second sensor 116 to stick to the skin of the patient 102. According to various implementations, the first sensor 114 is configured to detect the flow of blood in the heart of the patient 102. The second sensor 116, for instance, is configured to detect the flow of blood through a femoral artery of the patient 102.

In various implementations, it is advantageous for the rescuer 104 to monitor the ECG 110 and the flow of blood through at least a portion of the patient 102, simultaneously. However, it may be difficult for the rescuer 104 to visually monitor the ECG 110 and the flow of blood while also managing the medical emergency of the patient 102. In some cases, the first sensor 114 detects a velocity of blood through the heart (or a blood vessel) of the patient 102 over time and transmits, to the monitor-defibrillator 106, an indication of the velocity of the blood. The monitor-defibrillator 106, in various cases, generates and outputs an audio signal indicating the velocity of blood detected by the first sensor 114. For example, the monitor-defibrillator 106 may detect a pulse rate of the patient 102 by detecting peaks in the velocity of the blood over time. A pitch (i.e., frequency) of the audio signal may correspond to a difference between the detected pulse rate and a predetermined range. For example, the audio signal may increase in pitch if the pulse rate of the patient 102 falls below a lower threshold pulse rate. In various cases, the monitor-defibrillator 106 also visually outputs the ECG 110 on the display 112 while outputting the audio signal indicating the pulse rate of the patient 102. Accordingly, the rescuer 104 may monitor the pulse rate of the patient 102 without looking away from the ECG 110.

In some cases, the monitor-defibrillator 106 audibly reports an emergency condition detected by the first sensor 114 or the second sensor 116. For example, blood flow through the femoral artery of the patient 102 may cease due to the presence of a blood clot or other impediment to blood flow. The blood flow detected by the second sensor 116, for instance, falls below a threshold due to the blood clot or other impediment. In contrast, the blood flow through the heart of the patient 102, as detected by the first sensor 114, may remain above a threshold. In response to detecting hat the blood flow detected by the second sensor 116 is below a threshold, the monitor-defibrillator 106 may selectively output an audio signal based on the parameter detected by the second sensor 116, rather than the parameter detected by the first sensor 114. For example, the monitor-defibrillator 106, in various cases, may selectively output an audio signal indicating the absence of blood flow detected by the second sensor 116 and an indication of the second sensor 116 (e.g., the monitor-defibrillator 106 may output an audible cue instructing the rescuer 104 to check the leg of the patient 102 before outputting an audio signal indicating the real-time detected flow by the second sensor 116). In some cases, the monitor-defibrillator 106 mutes or otherwise ceases an audio signal based on the parameter detected by the first sensor 114. Accordingly, the monitor-defibrillator 106 may prioritize audible reporting of physiological parameters detected by different sensors based on importance.

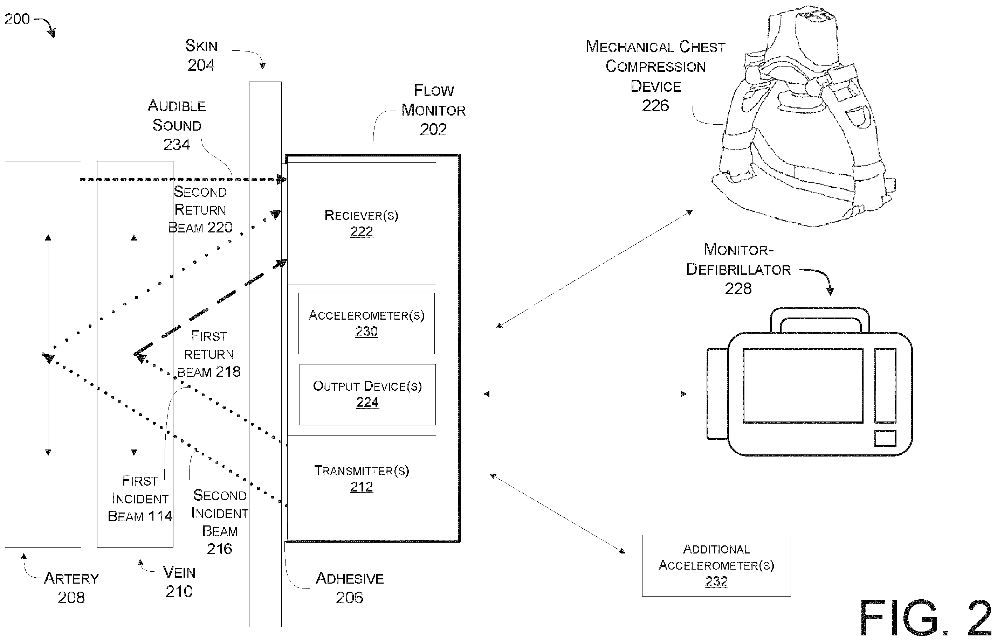

FIG. 2 illustrates an example environment 200 for monitoring blood flow through one or more blood vessels of a subject. A flow monitor 202 is adhered to skin 204 of the subject via an adhesive 206. In some examples, the flow monitor 202 includes the first sensor 114 and/or the second sensor 116. The flow monitor 202, for instance, is located outside of the body of the subject. In some implementations, the flow monitor 202 is held on the skin 204 by a strap, a buckle, a bandage, or some other fastener. For instance, the flow monitor 202 may be wrapped around an extremity of the subject.

In various implementations, an artery 208 and a vein 210 are disposed underneath the skin 204. The artery 208 and the vein 210 are part of the circulatory system of the subject. The circulatory system includes a fluid circuit of various blood vessels (including the artery 208 and the vein 210). The subject includes a heart that, when functioning, pumps blood through the blood vessels. In particular, the heart moves blood from lungs of the subject, where the blood can be oxygenated, to other portions of the subject's body, such as the brain, other organs, and the subject's extremities. Along the circulatory system, cells within the blood deliver oxygen to cells of the subject, thereby supporting cellular respiration. In various cases, the artery 208 carries oxygenated blood from the lungs of the subject and the vein 210 carries deoxygenated blood toward the lungs. Examples of the artery 208 include a carotid artery, a subclavian artery, a coronary artery, a brachial artery, an iliac artery, a radial artery, a femoral artery, or a pulmonary artery. Examples of the vein 210 include a jugular vein, an iliac vein, a subclavian vein, a cephalic vein, a brachial vein, a basilic vein, a hepatic vein, a radial vein, an ulnar vein, a digital vein, a brachiocephalic vein, a femoral vein, a saphenous vein, a venous arch, or a tibial vein. In some cases, the pulmonary artery carries deoxygenated blood.

According to various implementations of the present disclosure, the flow monitor 202 is configured to detect the flow of blood through the artery 208 and/or vein 210. In particular cases, the flow monitor 202 includes one or more transmitters 212 configured to output one or more incident beams toward the artery 208 and/or vein 210. In the example illustrated in FIG. 2, the incident beams include a first incident beam 214 and a second incident beam 216.

In various cases, the first incident beam 214 and the second incident beam 216 include waves that are transmitted through the skin 204. The waves, for example, can be instantiated as light and/or sound. In various cases, the first incident beam 214 and the second incident beam 216 include at least one of infrared, near-infrared, or visible light. For instance, the light may have a frequency in a range of 300 GHz - 230 THz and a wavelength in a range of 700 nanometers (nm) to 1 millimeter (mm). For instance, the transmitter(s) 212 include one or more light sources, such as light-emitting diodes (LEDs) or lasers. In some examples, the transmitter(s) 212 may include one or more mirrors configured to split the light output by the light source(s), such as for an interferometric analysis. In some cases, the receiver(s) 222 include one or more light sensors, such as at least one of a photodiode, a phototransistor, a photomultiplier tube, a charge-coupled device, a metal-semiconductor-metal photodetector, or a complementary metal oxide semiconductor photodetector.

According to various implementations, the waves include ultrasound. As used herein, the term "ultrasound," and its equivalents, can refer to mechanical waves (e.g., in the form of pressure waves) having a frequency in a range of 20 kilohertz (kHz) to 200 megahertz (MHz). Ultrasound, for example, is sound in a frequency that is greater than an upper detection limit of a human ear. In various instances, transmitter(s) 212 include one or more piezoelectric crystals (including, e.g., lead zirconate titanate (PZT), LiNbO₃ (LN), lead magnesium niobate-lead titanate (PMN-PT), or lead indium niobate- lead magnesium niobate-lead titanate (PIN-PMN-PT)). When an electrical current is induced through the piezoelectric crystal(s), the piezoelectric crystal(s) vibrate at a frequency that produces ultrasound. In some examples, the transmitter(s) 212 include one or more micro-electromechanical system (MEMS) devices. In some instances, the transmitter(s) 212 include one or more capacitive micromachined ultrasonic transducers (CMUTs) and/or piezoelectric micromachined ultrasonic transducers (PMUT). Any electrical to mechanical conversion system or material that operates at the ultrasound frequency range would be suitable.

According to various implementations, the flow monitor 202 includes one or more ultrasound transducers. As used herein, the terms "ultrasound transducer," "ultrasonic transducer," "transducer," and their equivalents, may refer to a device that generates or detects ultrasound. For instance, the ultrasound transducer(s) include the transmitter(s) 212 and/or the receiver(s) 222. In some cases, an ultrasound transducer includes a transducer element (e.g., a piezoelectric crystal, MEMS device, or another type of electrical-to-mechanical conversion device), a first electrode disposed on one side of the transducer element, and a second electrode disposed on another side of the transducer element. In various implementations, the ultrasound transducer is configured to produce ultrasound by inducing a current through or a voltage between the first and second electrodes. In some cases, the ultrasound transducer is configured to detect ultrasound by detecting a current through or voltage between the first and second electrodes that is induced when the ultrasound is received by the transducer element. In some cases, the ultrasound transducer is encased in a housing, which may be watertight. The ultrasound transducer, in some examples, further includes a matching layer that is disposed between the transducer element and a surface of the housing from which an incident beam of ultrasound is emitted. The matching layer includes a material having an acoustic impedance that is between the acoustic impedance of the transducer element and an acoustic lens (if one exists or between the transducer and skin). In some implementations, a gel layer is disposed between the housing of the ultrasound transducer and the skin 204 that further matches the impedance between the ultrasound transducer and the body, thereby preventing the first incident beam 214 and the second incident beam 216 from being reflected by an interface containing air between the skin 204 and the ultrasound transducer. In some cases, the adhesive 206 serves as the gel layer.

In some examples, the first incident beam 214 is reflected and/or scattered by blood in the vein 210, thereby generating a first return beam 218. Further, in some cases, the second incident beam 216 is reflected and/or scattered by blood in the artery 208, thereby generating a second return beam 220. The flow monitor 202 includes one or more receivers 222 configured to detect the first return beam 218 and/or the second return beam 220.

As illustrated, the artery 208 and the vein 210 are substantially parallel to the skin 204. In various implementations of the present disclosure, the transmitter(s) 212 emit the first incident beam 214 and/or the second incident beam 216 in a direction that is non-perpendicular and non-parallel to the surface of the flow monitor 202 that is adhered to the skin 204. That is, the transmitter(s) 212 emit the first incident beam 214 and/or the second incident beam 216 in an angled fashion. Thus, a component of the first incident beam 214 is parallel to the blood flow through the vein 210 and/or a component of the second incident beam 216 is parallel to the blood flow through the artery 208.

Various implementations of the transmitter(s) 212 that enable angled transmission of the first incident beam 214 and the second incident beam 216 are disclosed herein. In some implementations in which the transmitter(s) 212 include one or more transducer elements configured to emit ultrasound, an individual transducer element may have the shape of a cylinder (with a height of the cylinder being substantially parallel to the skin 204 and perpendicular to a direction of the artery 208 and/or vein 210) or a polygonal prism (with a height of the polygonal prism being substantially parallel to the skin 204 and perpendicular to a direction of the artery 208 and/or vein 210). In some cases, a piezoelectric crystal may be divided into sections defined by an angle perpendicular to the height of the cylinder, each section serving as a different transducer element configured to emit ultrasound. In some cases, the transmitter(s) 212 include multiple (e.g., planar) transducer elements that emit respective ultrasound beams that are phase networked together such that they collectively form the angled first incident beam 214 or the second incident beam 216. In some cases, the transmitter(s) 212 include a single transducer element including portions that are insensitive to ultrasound (e.g., non-piezoelectric portions, such as including a polymer) and portions that are sensitive to ultrasound (e.g., piezoelectric portions), which can cause the first incident beam 214 or the second incident beam 216 to be output as a grating lobe. The grating lobe may include components emitted at different angles, at least one of which may include the first incident beam 214 or the second incident beam 216 to be transmitted at an angle with respect to the artery 208 or vein 210.

In some implementations, the transmitter(s) 212 include a transducer element that emits ultrasound from a surface that is nonparallel to the skin 204. For example, the flow monitor 202, in some cases, includes a wedge-shaped spacer between the crystal and the skin 204. The spacer may be substantially acoustically transparent. In some examples, the wedge-shaped spacer includes a polymer or gel that is configured to perform impedance matching between the crystal and the skin 204. For instance, the spacer may include multiple layers arranged in steps that are configured to be in contact with the crystal and/or tilt the crystal with respect to the skin 204.

In some cases, the flow monitor 202 includes an acoustic "lens" that is disposed between a crystal emitting ultrasound and the skin 204. For example, the acoustic lens can be a spacer with a nonuniform acoustic impedance. Thus, the acoustic lens may bend the ultrasound emitted by the crystal before it is transmitted through the skin 204.

In some examples, the transmitter(s) 212 includes a crystal that is disposed inside of a needle that is disposed through the skin 204. In some implementations, the transmitter(s) 212 include an array of crystals configured to emit the first incident beam 214 and/or the second incident beam 216 through the skin 204. For example, the array may be arranged on a curved surface, such that individual crystals may point in different directions. In operation, the flow monitor 202 may automatically determine which incident beams whose return beams produce a greatest frequency and/or phase shift with respect to the incident beams, and may define those beams as the first incident beam 214 and/or the second incident beam 216.

In various implementations, the first return beam 218 may represent a frequency and/or phase shift with respect to the first incident beam 214 due to the Doppler effect. Similarly, because the component of the second incident beam 216 is parallel to the blood flow through the artery 208, the second return beam 220 may represent a frequency and/or phase shift with respect to the second incident beam 216 due to the Doppler effect. These shifts occur due to the Doppler effect and may be referred to as "Doppler shifts."

According to various implementations, the flow monitor 202 determines a velocity of the blood flow through the artery 208 and the vein 210 due to a difference between the frequencies of the first incident beam 214 and the first return beam 218, as well as a difference between the frequencies of the second incident beam 216 and the second return beam 220. The transmitter(s) 212, in some cases, operate in a continuous wave Doppler mode (also referred to as "CW Doppler"), and continuously transmit the first incident beam 214 and the second incident beam 216 during a monitoring period. The receiver(s) 222, for instance, continuously detect the first return beam 218 and the second return beam 220 during the monitoring period. For instance, the flow monitor 202 detects the blood velocity in the artery 208 and the blood velocity in the vein 210, in-real time, continuously or semi-continuously based on the frequency shifts between the first incident beam 214 and the first return beam 218 as well as between the second incident beam 216 and the second return beam 220.

In various implementations, the flow monitor 202 operates in a pulsed-wave Doppler mode (also referred to as "PW Doppler"). For instance, the flow monitor 202 causes the incident beam 216 to output the first incident beam 214 and/or the second incident beam 216 in pulses and detects the first return beam 218 and/or the second return beam 220 as return pulses. In various cases, a time delay between the output pulses and the return pulses is indicative of the depth of a structure from which the return pulses are reflected. In various implementations, the flow monitor 202 can determine the blood velocity in the artery 208 based on phase shifts between the pulses of the first incident beam 214 and the first return beam 218. Further, the flow monitor 202 can determine the blood velocity in the vein 210 based on phase shifts between the pulses of the second incident beam 216 and the second return beam 220.

In some cases, the flow monitor 202 detects the blood velocities at a sampling rate that corresponds to at least twice the component of the velocity range in the direction of the beam pointing angle of the first incident beam 214 and/or the second incident beam 216.

In some examples, the flow monitor 202 performs laser Doppler velocimetry in order to detect the blood velocity. In various cases, the flow monitor 202 includes one or more interferometric sensors. For example, the first incident beam 214 and the second incident beam 216 are light beams (e.g., coherent light beams) that are split (e.g., by one or more mirrors) prior to transmission through the skin 204. The flow monitor 202 may detect the blood velocities in the artery 208 and the vein 210 by comparing the first return beam 218 and the second return beam 220 to the split beams generated from the first incident beam 214 and the second incident beam 216. Techniques for interferometric detection of blood velocity can be found in, for example, R. D. Rader, C. M. Stevens and J. P. Meehan, "An Interferometric Blood Flow Measurement Technique - A Brief Analysis," in IEEE Transactions on Biomedical Engineering, vol. BME-21, no. 4, pp. 293-297, July 1974; Nagahara, et al., Method. Invest. Ophthalmol. Vis. Sci. 2011;52(1):87-92; and Robinson, et al., Sci Rep 13, 8803 (2023).

In some cases, the flow monitor 202 images a portion of the subject that includes the artery 208 and the vein 210. In some cases, the flow monitor 202 generates an image using the first return beam 218 and the second return beam 220 using one or more sonographic techniques. In various implementations, the flow monitor 202 generates the image using multiple return beams including the first return beam 218 and the second return beam 220. The flow monitor 202, in some implementations, generates the multiple return beams by sweeping the first incident beam 214 and the second incident beam 216 across a section of the subject being imaged. For example, the flow monitor 202 may generate a real-time image of the subject that includes cross-sections of the artery 208 and vein 210, respectively. In some implementations, the flow monitor 202 automatically segments the cross-sections of the artery 208 and vein 210 in the real-time image. In some cases, the flow monitor 202 performs segmentation of a scrolling Doppler image (e.g., Doppler shift in a y-axis is swept in time along an x-axis) to segregate out the Doppler information of the vein 210 from the artery 208. Various types of segmentation techniques can be used, such as detection using histogram of oriented gradients (HOG) features, a scale-invariant feature transform (SIFT), Viola-Jones object detection framework, or You Only Look Once (YOLO). Once the cross-sections depicted in the image are identified using image segmentation, the flow monitor 202 can further classify the cross-sections using a support vector machine (SVM). In some cases, the flow monitor 202 uses one or more trained convolutional neural networks (CNNs) to segment and/or classify the cross-sections of the artery 208 and the vein 210 depicted in the image. In various cases, the flow monitor 202 may differentiate the cross-section corresponding to the artery 208 an the cross-section corresponding to the vein 210.

In particular implementations, the flow monitor 202 detects the velocity of the blood through the artery 208 and the velocity of the blood through the vein 210, simultaneously. For example, the first return beam 218 may be reflected from the artery 208 and the second return beam 220 may be reflected from the vein 210, so that the flow monitor 202 can detect the blood velocity of the artery 208 based on the first return beam 218 and may detect the blood velocity of the vein 210 based on the second return beam 220.

Simultaneously monitoring the blood velocity through the artery 208 and the vein 210 may enable certain evaluations of the subject. In some implementations, the flow monitor 202 detects a volumetric flow rate or net flow volume (e.g., during a time period) through at least a portion of the subject based on the blood velocity through the artery 208 and the vein 210. In some cases, the flow monitor 202 determines the volumetric flow rate through the artery 208 by integrating the velocity of the blood through the artery 208 across a cross-sectional area of the artery 208. The flow monitor 202 may determine the volumetric flow rate through the vein 210 by integrating the velocity of the blood through the vein 210 across a cross-sectional area of the vein 210. In various implementations, the flow monitor 202 is configured to detect a net flow volume that flows through the cross-section of the artery 208 during a time period (e.g., a cardiac cycle, a chest compression cycle, a portion of a chest compression cycle, or any combination thereof) by integrating the volumetric flow rate through the artery 208 over the time period. Similarly, the flow monitor 202 is configured to detect a net flow volume that flows through the cross-section of the vein 210 during the time period by integrating the volumetric flow rate through the vein 210 over the time period.

The artery 208, for instance, supplies oxygenated blood to the portion of the subject, and the vein 210 transports deoxygenated blood from the portion of the subject. In some examples, the flow monitor 202 is configured to detect a net flow volume of blood to a portion of the subject's body over time. In particular cases, the artery 208 is a carotid artery and the vein 210 is a jugular vein, and the flow monitor 202 is configured to detect a net flow volume of blood to the brain of the subject over time.

The flow monitor 202 may assess a condition of the subject and/or evaluate a treatment administered to the subject by analyzing blood flow parameters (e.g., velocity, volumetric flow rate, or net flow volume of blood) in the artery 208 and/or the vein 210. In some implementations, the flow monitor 202 identifies chest compressions performed on the subject based on the blood velocity through the artery 208 and/or the blood velocity through the vein 210. When (e.g., effective) chest compressions are performed on the subject, the compressions push blood through the artery 208 and the vein 210 in a direction that moves distally from the chest. Thus, chest compressions cause blood flowing through the artery 208 and blood flowing through the vein 210 to travel in parallel directions. Moreover, an individual chest compression causes the blood to flow in the artery 208 and vein 210 in a surge. Thus, in some cases, the flow monitor 202 detects that chest compressions are being performed on the subject by detecting that the blood through the artery 208 and the blood through the vein 210 is traveling in the same or parallel directions (e.g., in a direction pointing distally from the chest). For instance, the flow monitor 202 can detect whether chest compressions are undesirably moving blood in the same direction through the artery 208 and the vein 210.

In some cases, the flow monitor 202 provides feedback about the efficacy of the chest compressions based on the blood velocity through the artery 208 and the vein 210 and/or the net flow volume to the portion of the subject. For instance, if the flow monitor 202 detects less than a threshold blood velocity through the artery 208 or vein 210, or detects less than a threshold net flow volume to the portion of the subject (e.g., during a particular time period), the flow monitor 202 can output a feedback signal via one or more output devices 224. The output device(s) 224, for instance, include a display (e.g., a screen configured to visually output signals), a speaker (e.g., configured to audibly output signals), a haptic feedback device (e.g., configured to convey signals by vibrating or otherwise moving), a transceiver (e.g., configured to transmit signals to external devices), or any combination thereof. In some cases, the feedback signal is output to a rescuer (not illustrated) performing the chest compressions, and may cause the rescuer to adjust the depth of the chest compressions, adjust the frequency of the chest compressions, or adjust the position of the chest compressions relative to the subject's chest.

In some implementations, the feedback signal is transmitted to a mechanical chest compression device 226 that is performing the chest compressions. The mechanical chest compression device 226, in some cases, administers the chest compressions by moving a plunger up and down on the subject's chest. The feedback signal, for instance, causes the mechanical chest compression device 226 to adjust the depth of the chest compressions, adjust the frequency of the chest compressions, adjust the position of the plunger relative to the subject's chest, pause chest compressions, or initiate chest compressions. In some implementations, the mechanical chest compression device 226 transmits a signal to the flow monitor 202 that indicates the timing, frequency, position, or another chest compression parameter characterizing the chest compressions administered by the mechanical chest compression device 226. The flow monitor 202, in some cases, generates the feedback signal based on the chest compression parameter, such that the feedback signal is specific to the conditions reported by the mechanical chest compression device 226.

The placement of chest compressions, in particular, impacts the flow of blood through specific blood vessels through the subject's body. In some cases, the flow monitor 202 enables optimization of the position of the chest compressions based on the flow rate through the artery 208, the flow rate through the vein 210, the net flow volume through the portion of the subject's body, a current position of the chest compressions, an identity or position of the artery 208 in the subject's body, an identity or position of the vein 210 in the subject's body, or any combination thereof. For example, if the chest compressions are being administered to the right of an ideal position, the flow monitor 202 may detect a peak and/or mean flow rate of blood in the artery 208 that is below a threshold. In various implementations, the flow monitor 202 (or another computing device that is communicatively coupled to the flow monitor 202) generates a feedback signal including an instruction to apply the chest compressions one direction or another and/or closer to the ideal position.

According to various implementations, the flow monitor 202 can detect the presence of spontaneous circulation of the subject, in which the heart of the subject is spontaneously pumping a sufficient amount of blood through the body of the subject. In some cases, the flow monitor 202 detects a return of spontaneous circulation (ROSC). The flow monitor 202, for instance, can detect spontaneous circulation while the subject is receiving chest compressions. As noted previously, when the subject is receiving chest compressions, the chest compressions cause parallel or unidirectional blood flow in the artery 208 and the vein 210. In contrast, the pumping heart of the subject circulating blood through the subject's circulatory system causes antiparallel or bidirectional blood flow in the artery 208 and the vein 210. Thus, the flow monitor 202, in some cases, detects spontaneous circulation in response to detecting antiparallel or bidirectional blood flow in the artery 208 and vein 210.

In some cases, the flow monitor 202 detects spontaneous circulation using other techniques. In general, the heart is capable of pumping blood at a greater peak velocity than chest compressions. Therefore, the monitor 202 may detect spontaneous circulation by detecting that a blood velocity through the artery 208 or the vein 210 is greater than a threshold. Further, the blood velocity through the artery 208 or the vein 210 with respect to time during chest compressions is different than the blood velocity through the artery 208 or the vein 210 with respect to time during spontaneous circulation. According to some implementations, the flow monitor detects spontaneous circulation by determining that at least one frequency component of the volumetric flow rate over time through the artery 208 or vein 210 has changed. For instance, a shape or morphology of the volumetric flow rate over time can be indicative of spontaneous circulation.

Certain functionalities of the flow monitor 202 are enhanced by communications with other devices, such as the mechanical chest compression device 226 and a monitor-defibrillator 228. Collectively, any combination of the flow monitor 202, the mechanical chest compression device 226, and the monitor-defibrillator 228 may be a resuscitation system that is configured to resuscitate a single subject. For example, the flow monitor 202 is configured to modify and/or temporally gate measurements it performs based on communications from the mechanical chest compression device 226 and/or the monitor-defibrillator 228. In some cases, the flow monitor 202 receives a communication signal from the mechanical chest compression device 226 that indicates a frequency of chest compressions administered by the mechanical chest compression device 226, a timing of chest compressions administered by the mechanical chest compression device 226, or a time of a pause in the chest compressions administered by the mechanical chest compression device 226. In some cases, the flow monitor 202 is configured to remove a chest compression artifact from a blood velocity over time based on the communication signal from the mechanical chest compression device 226. For instance, the flow monitor 202 may remove an artifact from the blood velocity by applying a band reject filter centered around the chest compression frequency or by applying a comb filter that includes the chest compression frequency and one or more harmonics of the chest compression frequency. In some cases, the flow monitor 202 is configured to temporally gate a blood velocity measurement during a time window in which the mechanical chest compression device 226 has paused chest compressions or between chest compressions performed by the chest compression device 226. Using these techniques, in some cases, may enable the flow monitor 202 to more accurately identify spontaneous circulation of the subject by distinguishing between blood flow caused by the chest compressions and spontaneous blood flow induced by the heart of the subject.

In some cases, the monitor-defibrillator 228 transmits a communication signal to the flow monitor 202 indicating a time at which the monitor-defibrillator 228 is administering a treatment to the subject, such as an electrical shock or pace pulses. Because the electrical shock or pace pulses can interfere with the accuracy of other measurements performed by the flow monitor 202, the flow monitor 202 may gate the measurements at a time interval that omits the treatment to the subject. In some implementations, the flow monitor 202 includes sensitive electronics that could potentially be damaged when the treatment is administered to the subject. According to some examples, the flow monitor 202 includes a circuit with at least one switch that disconnects or otherwise shields the sensitive electronics during the treatment.

In some examples, the flow monitor 202 includes or is otherwise communicatively coupled to devices that include one or more sensors (not illustrated) configured to detect other physiological parameters. For example, the flow monitor 202 includes or is communicatively coupled with a sensor configured to detect an electrocardiogram (ECG), an oximetry sensor (e.g., a regional oximetry sensor, a pulse oximetry sensor, a cerebral oximetry sensor, or the like), or both.

In various implementations, the flow monitor 202 includes one or more accelerometers 230 configured to detect an acceleration of the flow monitor 202 and/or the skin 204 of the subject. The acceleration, for instance, is indicative of chest compressions administered to the subject or a pulse of the subject through the artery 208 or vein 210. Thus, in some cases, the flow monitor 202 detects whether the subject has a pulse based on the acceleration detected by the accelerometer(s) 230. At least one additional accelerometer 232 may further be disposed on another portion of the subject (e.g., the subject's chest) and may be configured to detect an acceleration indicative of the chest compressions. The flow monitor 202, in some implementations, removes a chest compression artifact of the acceleration detected by the accelerometer(s) 230 over time based on the acceleration detected by the additional accelerometer(s) 232 over time. In various implementations, the flow monitor 202 is configured to accurately detect spontaneous circulation or another condition of the subject based on the detected accelerations.

As used herein, the term "measurement" may refer to a blood velocity, a net blood flow volume, an ECG, an oxygenation of the subject's blood, an acceleration, or another physiological parameter of the subject. In some cases, the flow monitor 202 validates and/or temporally gates a first measurement in view of a second measurement. For example, if the ECG indicates that the heart of the subject is beating, but the blood velocity or net blood flow indicates that oxygenated blood is not circulating in the subject, then the flow monitor 202 may refrain from indicating that the subject has spontaneous circulation. In some cases, the flow monitor 202 may output a signal indicating that the subject has pulseless electrical activity (PEA).

In various cases, the flow monitor 202 is configured to detect blood flow in the brain of the subject. In some cases, the artery 208 is a part of the circle of Willis of the subject. If the subject is an adult, the skull of the subject may highly attenuate the first incident beam 214, the second incident beam 216, the first return beam 218, and the second return beam 220. For instance, the skull may have high attenuation with respect to ultrasound and/or light (e.g., at certain frequencies). Thus, in various examples, the flow monitor 202 is configured to direct the first incident beam 214, the second incident beam 216, the first return beam 218, and the second return beam 220 through a window in the skull. For instance, the flow monitor 202 is configured to monitor the artery 208 and/or vein 210 through a temple of the subject, an orbital socket of the subject, or an ear canal of the subject. In some cases, the flow monitor 202 is strapped to the head of the subject, such as in the form of an eyepatch.

In some cases, the operation of the flow monitor 202 is optimized in other ways to enable monitoring within the brain. For instance, if the first incident beam 214 and the second incident beam 216 include ultrasound, the ultrasound may have a frequency of less than 1 MHz. Although sub MHz ultrasound is incapable of generating high-resolution images, in some cases, the flow monitor 202 detects the flow through the artery 208 or vein 210 without generating an image of the artery 208 or vein 210. That is, sub MHz ultrasound is sufficient for Doppler flow detection in various implementations described herein.

Another way in which the flow monitor 202 is optimized for monitoring with the brain relates to the type of the artery 208 and/or vein 210 monitored by the flow monitor 202. Attenuation of an incident beam increases as it travels through an attenuating structure. Thus, in some cases, a distance between the flow monitor 202 (e.g., disposed on the skin 204 or eye) is less than a threshold distance.

In particular cases, it may be difficult to differentiate specific blood vessels in the brain (e.g., within the Circle of Willis) in which the first return beam 218 and/or the second return beam 220 are reflected and/or scattered. Further, it may be difficult to identify angles from which the blood vessels in the brain are disposed with respect to the flow monitor 202. Accordingly, it may be difficult to accurately quantify blood flow parameters of a blood vessel in the brain using techniques described herein. However, even detecting the presence or absence of movement of blood in the brain can provide helpful feedback in order to detect chest compression efficacy and/or spontaneous circulation. According to various implementations, the flow monitor 202 outputs an indication if the flow monitor 202 detects movement, or the absence of movement, or the relative difference of movement between the compression and decompression parts of CPR or the diastolic and systolic parts of a normal sinus rhythm of blood in the brain of the subject.

By detecting blood flow in the brain, the flow monitor 202 is configured to detect an efficacy of chest compressions being administered to the subject. Technologies that monitor blood flow in extremities can indirectly detect chest compression efficacy. However, because a primary purpose of chest compressions is to provide oxygenated blood to the brain of the subject, the flow monitor 202 is able to directly detect the efficacy of the chest compressions by monitoring blood flow in the brain. The flow monitor 202, for instance, can output a feedback signal (e.g., to the mechanical chest compression device 226 or to the user) indicating whether chest compressions are generating blood flow in the brain. In some cases, the feedback signal includes a metric indicative of the blood flow in the brain (e.g., a quantity that increases with an increase in a detected blood velocity or flow rate).

According to some examples, the receiver(s) 222 include one or more microphones configured to detect an audible sound 234 from the subject. For example, the flow monitor 202 may include the functionality of a sophisticated stethoscope. In some cases, the microphone(s) detect the audible sound 234 emitted by blood flowing through the artery 208 or vein 210. In some examples, the microphone(s) detect the audible sound 234 emitted by the heart of the subject. In various implementations, the audible sound 234 can be in an audible or inaudible range. The flow monitor 202, in various cases, detects the audible sound 234 at a sampling frequency, such as a sampling frequency of greater than 100 Hz, 1 kHz, or 10 kHz. In some implementations, the flow monitor 202 determines a condition of the subject based on the audible sound 234.

According to some cases, the output device(s) 224 include a speaker that outputs an audible signal indicative of the audible sound 234 detected by the flow monitor 202. For example, the output device(s) 224 output the sampled audible sound 234 into an audio headset of the user, or in an environment in which the user is present, at a volume that can be perceived by the user in an emergency scene. That is, the output device(s) 224 may amplify the audible sound 234. Some monitors output computer-generated sounds indicative of a heart rate of a subject, such as a "beep" sound whenever a QRS complex is detected in an ECG. According to some implementations, the audible sound 234 includes more diagnostic-relevant information that can be perceived by the user than a computerized "beep" sound. In some examples, a condition of the subject can be identified using the strength, velocity, or morphological characteristics of blood flow, but these features cannot necessarily be identified using audio output from a previous type of monitor. Furthermore, by outputting an amplified version of the audible sound 234 as an audible signal, the flow monitor 202 may convey potentially important information about the condition of the subject without visually outputting it on a display. The user, for example, may be monitoring other visual signals on the monitor-defibrillator 228, and thus the flow monitor 202 may enable cognitive offloading for the user.

In some implementations, the receiver(s) 222 include multiple microphones configured to detect audible sounds 234 from multiple locations on the subject's body. For instance, the microphones may detect the audible sound 234 from a right quadrant of the subject's chest and another audible sound 234 from a left quadrant of the subject's chest. The flow monitor 202, in some cases, analyzes the detected sounds for features indicative of a medical condition. For example, the flow monitor 202 detects the medical condition by detecting an anomaly in the detected sound. In some implementations, the flow monitor 202 selectively outputs a single one of the audible sounds based on an input signal received from the user. In some cases, the flow monitor 202 selectively outputs a single one of the audible sounds that is indicative of the medical condition. In various implementations, the output device(s) 224 are configured to output a signal indicating the medical condition.

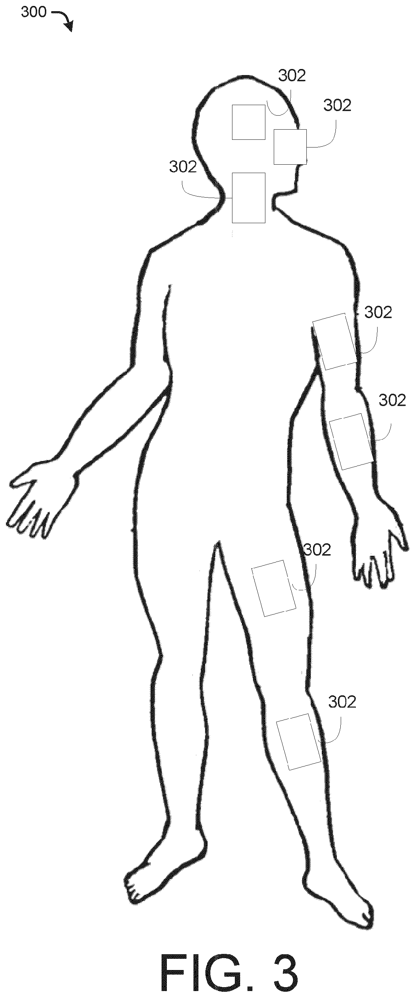

FIG. 3 illustrates various placements of a flow monitor 302 on the body of a subject 300. In some cases, the flow monitor 302 includes at least one of the first sensor 114, the second sensor 116, or the flow monitor 202. In various implementations, the flow monitor 302 may be disposed on a temple, an orbital socket, an ear canal, a neck, an upper arm, a lower arm, an upper leg, or a lower leg of the subject 300. In some implementations, the flow monitor 302 includes multiple physical devices that are disposed on different parts of the body of the subject 300, simultaneously. In various implementations, the flow monitor 302 may be integrated into a cot or other support on which the subject 300 is disposed.

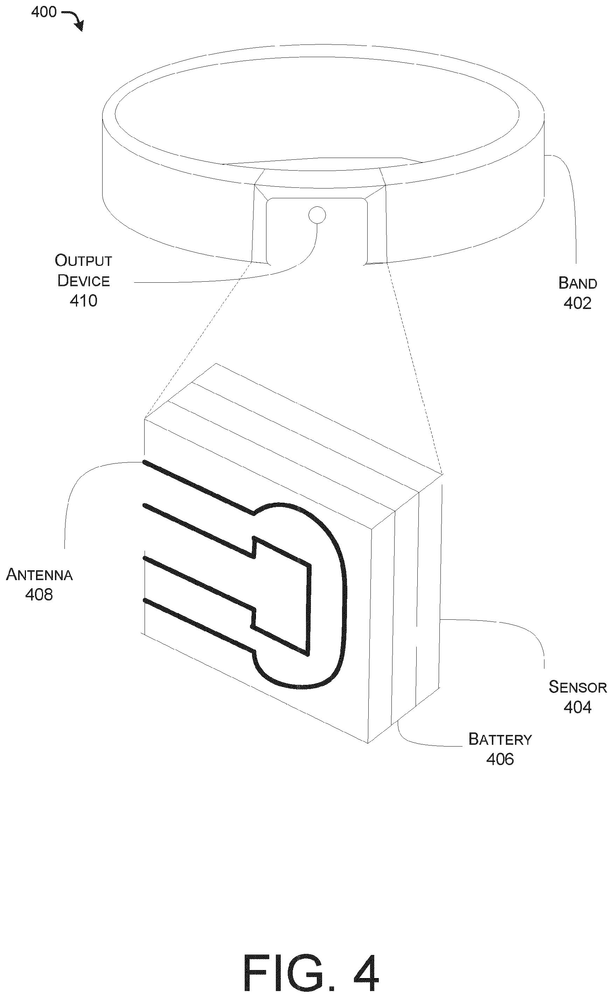

FIG. 4 illustrates the structure of an example flow monitor 400. The flow monitor 400, for instance, includes at least one of the first sensor 114, the second sensor 116, the flow monitor 202, or the flow monitor 302. In some cases, the flow monitor 400 is a patch that can be strapped to a subject or adhered to the skin of the subject. For instance, the flow monitor 400 includes a band 402 configured to be disposed around an appendage of the subject. According to some examples, the band 402 is configured to be disposed around a chest, abdomen, head, or neck of the subject.

In various implementations, the flow monitor 400 includes a sensor 404 configured to detect a physiological parameter indicative of blood flow through a subject. In some cases, the sensor 404 includes a microphone configured to detect a sound generated by the body of the subject. In some examples, the sensor 404 includes an ultrasound transducer configured to detect the velocity of blood flowing through at least one blood vessel of the subject and/or through the heart of the subject. In some examples, the sensor 404 includes an ultrasound transducer configured to detect heart wall motion. In some cases, the sensor 404 includes at least one transmitter and/or at least one receiver. In some examples, the sensor 404 includes one or more accelerometers.

The sensor 404, for instance, is powered by a battery 406. In some cases, the battery 406 is disposable. In some examples, the battery 406 is rechargeable.

The flow monitor 400, in various cases, further includes an antenna 408. The antenna 408, in various implementations, is configured to transmit and/or receive communication signals from an external device. In some cases, the antenna 408 enables the flow monitor 400 to communicate with another flow monitor or another type of medical device (e.g., an AED or monitor-defibrillator). For instance, the flow monitor 400 is configured to transmit, to an external device, an indication of the physiological parameter detected by the sensor 404. The antenna 408, in various cases, is powered by the battery 406.

According to various examples, the sensor 404, the battery 406, and the antenna 408 are arranged in respective layers of the flow monitor 400. For example, each layer may correspond to a circuit board (e.g., a PCB) that accommodates a respective component. In various cases, the layer corresponding to the sensor 404 is configured to be adjacent to the subject when the flow monitor 400 is disposed on the subject. The layer corresponding to the antenna 408, for example, faces away from the subject when the flow monitor 400 is disposed on the subject. Accordingly, the body of the subject may be prevented from interfering with wireless signals transmitted and/or received by the antenna 408.

In some examples, the flow monitor 400 further includes an output device 410. The output device 410 is configured to output a signal to a user. In some cases, the signal is based on the physiological parameter detected by the sensor 404. For instance, the output device 410 includes a light that is activated when a blood velocity detected by the sensor 404 remains below a threshold blood velocity for greater than a threshold period of time.

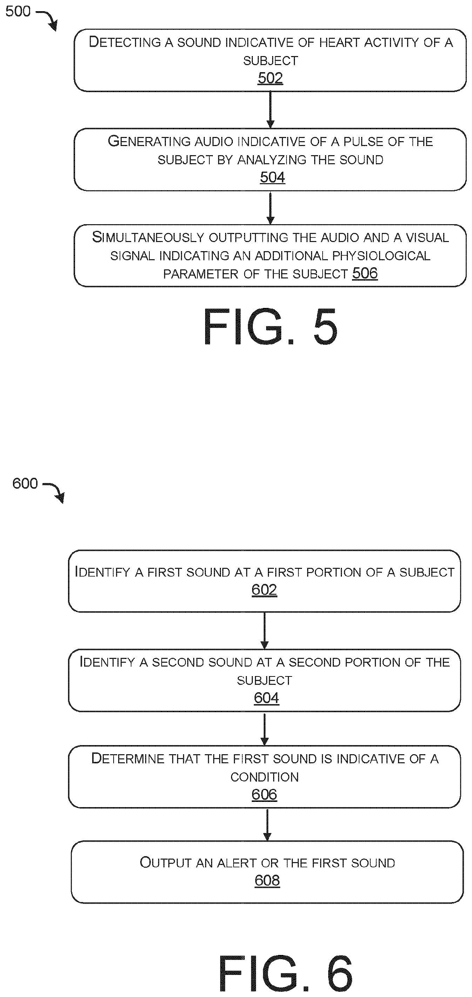

FIGS. 5 to 7 illustrate various processes related to implementations of the present disclosure. Although steps in the processes are illustrated as being performed in a particular order, implementations are not necessarily limited to the orders illustrated. Further, in some cases, the processes may be performed multiple times (e.g., periodically).

FIG. 5 illustrates an example process 500 for conveying physiological parameters using both audio and a visual signal. In various cases, the process 500 is performed by an entity including a sensor (e.g., the first sensor 114, the second sensor 116, or the like), a flow monitor (e.g., the flow monitor 202, the flow monitor 302, or the like), a medical device (e.g., the monitor-defibrillator 106, the mechanical chest compression device 226, the monitor-defibrillator 228, the additional accelerometer(s) 232, the flow monitor 400, or the like), at least one processor, at least one computing device, or any combination thereof.

At 502, the entity detects a sound indicative of heart activity of a subject. The sound, for instance, is generated by a movement of the heart of the subject, by blood flowing through at least one blood vessel of the subject, or both. In some cases, the sound includes a reflection of an incident beam from the heart of the subject and/or from a blood vessel of the subject. For example, the incident beam includes ultrasound. According to some implementations, the entity transmits the incident beam toward the heart and/or blood vessel of the subject.

At 504, the entity generates audio indicative of a pulse of the subject by analyzing the sound. According to various cases, the entity uses Doppler-based techniques to identify the velocity of a heart wall and/or blood of the subject. For example, the entity compares a frequency of the reflection of the incident beam to the frequency of the incident beam. The difference between the frequency of the reflection of the incident beam and the frequency of the incident beam, in various cases, is indicative of the velocity of the heart wall and/or blood that has reflected the incident beam. In some cases, the entity generates the audio based on the detected velocity. For example, the entity generates audio whose pitch and/or volume is proportional to the velocity of the heart wall and/or blood. In some cases, the entity compares a metric associated with the sound (e.g., a pitch, magnitude of a spectral component, velocity, etc.) to a reference value. The pitch and/or volume of the audio, in some cases, is proportional to a difference between the metric and the reference value.

At 506, the entity simultaneously outputs the audio and the visual signal. The visual signal indicates an additional physiological parameter of the subject. For example, the entity detects at least one of an ECG, a plethysmograph, a capnograph, or some other physiological parameter over time. In some cases, the visual signal represents a waveform of the additional physiological parameter over time. For instance, the entity displays the visual signal on a screen.

According to some examples, the entity outputs the audio via at least one speaker. In various cases, the entity outputs the audio substantially in real-time as the sound indicative of the heart activity is being detected. For example, the entity may detect the sound at a sampling frequency (e.g., in a range of 10 Hz to 10 kHz). In various cases, the entity adjusts the pitch and/or volume of the audio based on a sampled sound within a limited time delay (e.g., no more than 1 second, 0.1 seconds, or 0.001 seconds) of the sound being detected.

FIG. 6 illustrates an example process 600 for conveying a condition of a subject based on a detected sound. In various cases, the process 600 is performed by an entity including a sensor (e.g., the first sensor 114, the second sensor 116, or the like), a flow monitor (e.g., the flow monitor 202, the flow monitor 302, or the like), a medical device (e.g., the monitor-defibrillator 106, the mechanical chest compression device 226, the monitor-defibrillator 228, the additional accelerometer(s) 232, the flow monitor 400, or the like), at least one processor, at least one computing device, or any combination thereof.

At 602, the entity identifies a first sound at a first portion of a subject. In some cases, the entity detects the first sound. In some examples, the entity receives data indicative of the first sound from a separate sensor device. The first portion of the subject, for instance, includes a heart, a first lung, an airway, an abdomen, a chest, a first limb, a neck, a first organ, or a head of the subject. The first sound, in some cases, includes ultrasound. For example, the first sound may be the reflection of a first incident beam from the first portion of the subject.

At 604, the entity identifies a second sound at a second portion of the subject. In some cases, the entity detects the second sound. In some examples, the entity receives data indicative of the second sound from a separate sensor device. The second portion of the subject, for instance, includes the heart, a second lung, the airway, the abdomen, the chest, a second limb, the neck, a second organ, or the head of the subject. The second sound, in some cases, includes ultrasound. For example, the second sound may be the reflection of a second incident beam from the second portion of the subject. In various cases, the second portion of the subject is different than the first portion of the subject.

At 606, the entity determines that the first sound is indicative of a condition. In various cases, the condition includes a cardiac arrhythmia, an abnormal breath sound, an airway obstruction, a myocardial infarction, an absence of a pulse, internal bleeding, or any combination thereof. According to some implementations, the entity determines that the first sound is indicative of the condition by determining a characteristic of data indicative of the first sound. For example, the characteristic may include, or at least be based on, a magnitude and/or pitch of the first sound. In some cases, the entity determines that the first sound is indicative of the condition by comparing the characteristic to at least one threshold.

According to some cases, the characteristic includes a rate of peaks in the volume of the first sound. For instance, a rate of local peaks in the volume of a sound indicative of blood flow or heart wall movement may correspond to the pulse of the subject. In some cases, the entity detects a cardiac arrhythmia by determining that the pulse is above an upper threshold or that the pulse is below a lower threshold. In some examples, the entity determines that the subject has an absence of a pulse in response to detecting an absence of peaks in the volume of the sound over time.

In some cases, the characteristic includes a magnitude of a component of the first sound. For instance, the entity determines a magnitude of a particular frequency component (e.g., a component of the first sound in the frequency domain). The entity, in some examples, detects the condition by comparing the magnitude to at least one threshold. Certain abnormal breath sounds can be identified based on a particular frequency component of a sound generated by at least one lung of the subject. For instance, stridor can be detected if a magnitude of a frequency component of the first sound defined between 380 to 480 Hz is above a predetermined threshold.

At 608, the entity outputs an alert or the first sound. In some cases, the entity outputs a visual or auditory alert. For instance, the alert may indicate the location of the first portion of the subject. In some cases, the entity outputs audio indicative of the first sound. According to various implementations, the entity outputs the first sound itself. A user may therefore be informed of the condition.

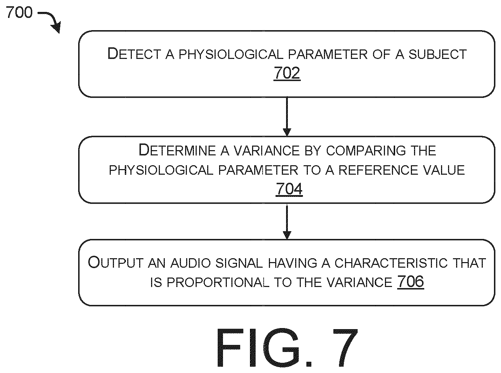

FIG. 7 illustrates an example process 700 for indicating a severity of a condition of a subject by outputting an audio signal. In various cases, the process 700 is performed by an entity including a sensor (e.g., the first sensor 114, the second sensor 116, or the like), a flow monitor (e.g., the flow monitor 202, the flow monitor 302, or the like), a medical device (e.g., the monitor-defibrillator 106, the mechanical chest compression device 226, the monitor-defibrillator 228, the additional accelerometer(s) 232, the flow monitor 400, or the like), at least one processor, at least one computing device, or any combination thereof.

At 702, the entity detects a physiological parameter of a subject. For example, the entity detects at least one of a sound of at least a portion of the subject, an acceleration of at least a portion of the subject, a blood pressure (e.g., non-invasive or invasive blood pressure), a blood flow (e.g., blood velocity) through at least one blood vessel and/or heart of the subject, a peak blood flow, an airway parameter (e.g., a respiration rate, a capnograph, an end-tidal carbon dioxide, an end-tidal oxygen, a partial pressure of carbon dioxide, or a partial pressure of oxygen), a plethysmograph, a blood oxygenation (e.g., a regional oxygenation or a pulse oxygenation), a heart rate, a pulse rate, a temperature (e.g., a core temperature), or a combination thereof. According to some examples, the entity determines a maximum or minimum of a detected physiological parameter during a time interval (e.g., a cardiac cycle, a ventilation cycle, or the like). For instance, the entity determines a peak (e.g., a maximum) of the physiological parameter during the time interval. In some cases, the entity detects the physiological parameter using a sensor, such as an accelerometer, an ultrasound transducer, a microphone, an infrared sensor, a blood pressure sensor (e.g., a blood pressure cuff or ultrasound-based blood pressure sensor), a blood oxygenation sensor (e.g., at least one photosensor configured to detect light transmitted and/or scattered from a portion of the body of the patient 102 from at least one light source, the transmitted and/or scattered light being indicative of an amount of oxygen bound to hemoglobin in the blood of the patient 102), a flow sensor, one or more electrodes, a gas sensor (e.g., a carbon dioxide or oxygen sensor), or any combination thereof.

At 704, the entity determines a variance by comparing the physiological parameter to a reference value. In some cases, the variance represents a difference between a metric associated with the physiological parameter and the reference value. Examples of the metric include a magnitude of the physiological parameter, a magnitude of a frequency component of the physiological parameter, a frequency of the physiological parameter, or the like. According to some cases, the reference value is based on a previous sample of the physiological parameter. For instance, the reference value may be a value of the physiological parameter detected during a first cardiac cycle of the subject, and the physiological parameter detected at 702 may be a value detected during a second cardiac cycle of the subject.

At 706, the entity outputs an audio signal having a characteristic that is proportional to the variance. The characteristic, for instance, includes a pitch or volume of the audio signal. In some cases, the audio signal includes at least two tones, wherein the characteristic corresponds to a dissonance (e.g., discordance) between the at least two tones. Thus, a dissonance of the audio signal may increase as the variance between the physiological parameter and the reference value increases. As used herein, the term "dissonance," and its equivalents, may refer to a complexity of a ratio between frequencies of at least two sounds. For example, two sounds may have low dissonance if their frequency ratio is an integer. However, two sounds may have high dissonance if their frequency ratio is represented by a non-integer. In some implementations, two sounds have low dissonance if they represent an octave, unison, perfect fourth, perfect fifths, or harmonics. In some cases, two sounds have high dissonance if they represent tritones, minor seconds, or major sevenths.

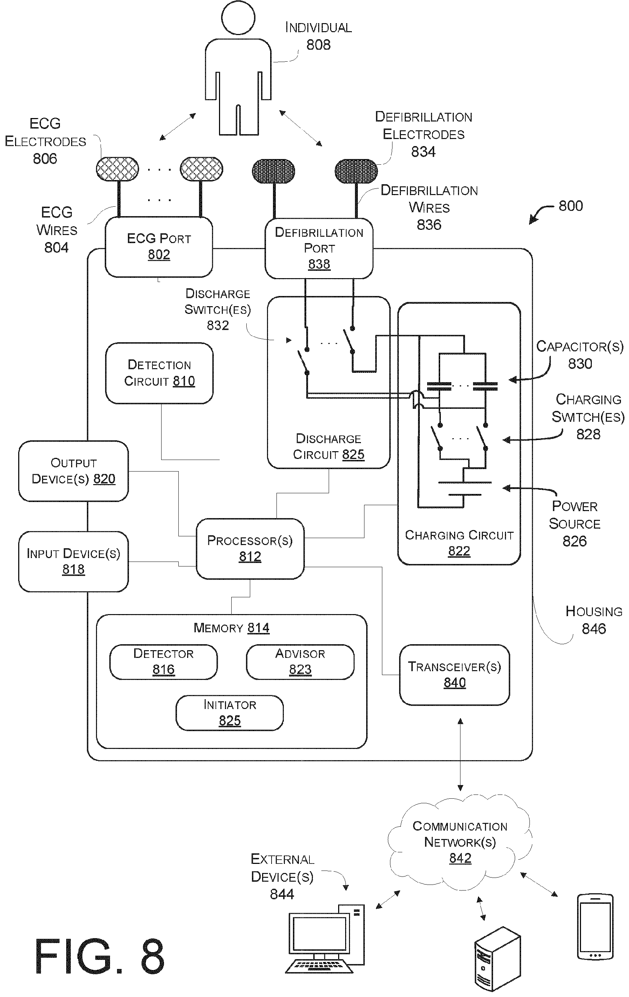

FIG. 8 illustrates an example of an external defibrillator 800 configured to perform various functions described herein. For example, the external defibrillator 800 is the monitor-defibrillator 228 described above with reference to FIG. 2.

The external defibrillator 800 includes an electrocardiogram (ECG) port 802 connected to multiple ECG wires 804. In some cases, the ECG wires 804 are removeable from the ECG port 802. For instance, the ECG wires 804 are plugged into the ECG port 802. The ECG wires 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 810 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 5-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG wires 804. In some cases, the detection circuit 810 includes one or more analog filters configured to filter noise and/or artifact from the electrical signals. The detection circuit 810 includes an analog-to-digital converter (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal one or more processors 812 in the external defibrillator 800. In some implementations, the processor(s) 812 includes a central processing unit (CPU), a graphics processing unit (GPU), digital signal processing unit (DPU), other processing unit or component known in the art, or any combination thereof.

The processor(s) 812 is operably connected to memory 814. In various implementations, the memory 814 is volatile (such as random access memory (RAM)), non-volatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and ventricular tachycardia (VT). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a switch, a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, a light indicator such as an LED, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 823, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 825 which is configured to, when executed by the processor(s) 812, cause the processor(s) 812 to control other elements of the external defibrillator 800 in order to administer a defibrillation shock to the individual 808. In some examples, the processor(s) 812 executing the discharge circuit 824 is configured to selectively cause the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

In various implementations, the memory 814 stores one or more components including instructions that, when executed by the processor(s) 812, cause the processor(s) 812 to perform one or more functions described herein. For example, the memory 814 may store instructions for performing at least one of the processes 500, 600, or 700.

The processor(s) 812 is operably connected to a charging circuit 822 and a discharge circuit 824. In various implementations, the charging circuit 822 includes a power source 826, one or more charging switches 828, and one or more capacitors 830. The power source 826 includes, for instance, a battery. The processor(s) 812 initiates a defibrillation shock by causing the power source 826 to charge at least one capacitor among the capacitor(s) 830. For example, the processor(s) 812 activates at least one of the charging switch(es) 828 in the charging circuit 822 to complete a first circuit connecting the power source 826 and the capacitor to be charged. Then, the processor(s) 812 causes the discharge circuit 824 to discharge energy stored in the charged capacitor across a pair of defibrillation electrodes 834, which are in contact with the individual 808. For example, the processor(s) 812 deactivates the charging switch(es) 828 completing the first circuit between the capacitor(s) 830 and the power source 826, and activates one or more discharge switches 832 completing a second circuit connecting the charged capacitor 830 and at least a portion of the individual 808 disposed between defibrillation electrodes 834.

The energy is discharged from the defibrillation electrodes 834 in the form of a defibrillation shock. For example, the defibrillation electrodes 834 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or VT) through the heart. In some examples, the defibrillation shock is 200 J or greater with a duration of about 0.015 seconds. In some cases, the defibrillation shock has a multiphasic (e.g., biphasic) waveform. The discharge switch(es) 832 are controlled by the processor(s) 812, for example. In various implementations, the defibrillation electrodes 834 are connected to defibrillation wires 836. The defibrillation wires 836 are connected to a defibrillation port 838, in implementations. According to various examples, the defibrillation wires 836 are removable from the defibrillation port 838. For example, the defibrillation wires 836 are plugged into the defibrillation port 838.

In various implementations, the processor(s) 812 is operably connected to one or more transceivers 840 that transmit and/or receive data over one or more communication networks 842. For example, the transceiver(s) 840 includes a network interface card (NIC), a network adapter, a local area network (LAN) adapter, or a physical, virtual, or logical address to connect to the various external devices and/or systems. In various examples, the transceiver(s) 840 includes any sort of wireless transceivers capable of engaging in wireless communication (e.g., radio frequency (RF) communication). For example, the communication network(s) 842 includes one or more wireless networks that include a 3^{rd} Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 840 includes other wireless modems, such as a modem for engaging in WI-FI^{®}, WIGIG^{®}, WIMAX^{®}, BLUETOOTH^{®}, or infrared communication over the communication network(s) 842.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 844 via the communication network(s) 842. The external devices 844 include, for instance, mobile devices (e.g., mobile phones, smart watches, etc.), Internet of Things (IoT) devices, medical devices (e.g., a flow monitor), computers (e.g., laptop devices, servers, etc.), or any other type of computing device configured to communicate over the communication network(s) 842. In some examples, the external device(s) 844 is located remotely from the defibrillator 800, such as at a remote clinical environment (e.g., a hospital). According to various implementations, the processor(s) 812 causes the transceiver(s) 840 to transmit data to the external device(s) 844. In some cases, the transceiver(s) 840 receives data from the external device(s) 844 and the transceiver(s) 840 provide the received data to the processor(s) 812 for further analysis.

In various implementations, the external defibrillator 800 also includes a housing 846 that at least partially encloses other elements of the external defibrillator 800. For example, the housing 846 encloses the detection circuit 810, the processor(s) 812, the memory 814, the charging circuit 822, the transceiver(s) 840, or any combination thereof. In some cases, the input device(s) 818 and output device(s) 820 extend from an interior space at least partially surrounded by the housing 846 through a wall of the housing 846. In various examples, the housing 846 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 830, discharges the capacitor(s) 830, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

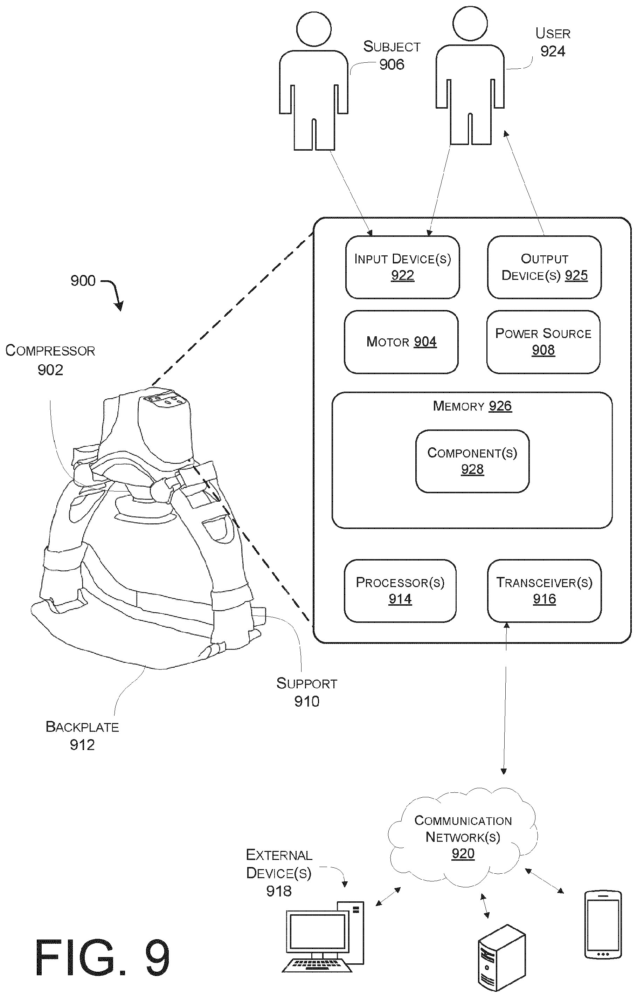

FIG. 9 illustrates a chest compression device 900 configured to perform various functions described herein. For example, the chest compression device 900 is the mechanical chest compression device 426 described with reference to FIG. 4.

In various implementations, the chest compression device 900 includes a compressor 902 that is operatively coupled to a motor 904. The compressor 902 is configured to physically administer a force to the chest of a subject 906 that compresses the chest of the subject 906. In some examples, the compressor 902 includes at least one piston that periodically moves between two positions (e.g., a compressed position and a release position) at a compression frequency. For example, when the piston is positioned on the chest of the subject 906, the piston compresses the chest when the piston is moved into the compressed position. A suction cup may be positioned on a tip of the piston, such that the suction cup contacts the chest of the subject 906 during operation. In various cases, the compressor 902 includes a band that periodically tightens to a first tension and loosens to a second tension at a compression frequency. For instance, when the band is disposed around the chest of the subject 906, the band compresses the chest when the band tightens.

The motor 904 is configured to convert electrical energy stored in a power source 908 into mechanical energy that moves and/or tightens the compressor 902, thereby causing the compressor 902 to administer the force to the chest of the subject 906. In various implementations, the power source 908 is portable. For instance, the power source 908 includes at least one rechargeable (e.g., lithium-ion) battery. In some cases, the power source 908 supplies electrical energy to one or more elements of the chest compression device 900 described herein.

In various cases, the chest compression device 900 includes a support 910 that is physically coupled to the compressor 902, such that the compressor 902 maintains a position relative to the subject 906 during operation. In some implementations, the support 910 is physically coupled to a backplate 912, cot, or other external structure with a fixed position relative to the subject 906. According to some cases, the support 910 is physically coupled to a portion of the subject 906, such as wrists of the subject 906.

The operation of the chest compression device 900 may be controlled by at least one processor 914. In various implementations, the motor 904 is communicatively coupled to the processor(s) 914. Specifically, the processor(s) 914 is configured to output a control signal to the motor 904 that causes the motor 904 to actuate or otherwise adjust the compressor 902. For instance, the motor 904 causes the compressor 902 to administer the compressions to the subject 906 based on the control signal. In some cases, the control signal indicates one or more treatment parameters of the compressions. Examples of treatment parameters include a frequency, timing, depth, force, position, velocity, and acceleration of the compressor 902 administering the compressions. According to various cases, the control signal causes the motor 904 to cease compressions, such as after ROSC has been detected.

In various implementations, the chest compression device 900 includes at least one transceiver 916 configured to communicate with at least one external device 918 over one or more communication networks 920. Any wired and/or wireless communication network described herein can be included in the communication network(s) 920 illustrated in FIG. 9. The external device(s) 918, for example, includes at least one of a flow monitor, a monitor-defibrillator, an AED, an ECMO device, a ventilation device, a subject monitor, a mobile phone, a server, or a computing device. In some implementations, the transceiver(s) 916 is configured to communicate with the external device(s) 918 by transmitting and/or receiving signals wirelessly. For example, the transceiver(s) 916 includes a NIC, a network adapter, a LAN adapter, or a physical, virtual, or logical address to connect to the various external devices and/or systems. In various examples, the transceiver(s) 916 includes any sort of wireless transceivers capable of engaging in wireless communication (e.g., RF communication). For example, the communication network(s) 920 includes one or more wireless networks that include a 3GPP network, such as an LTE RAN (e.g., over one or more LTE bands), an NR RAN (e.g., over one or more NR bands), or a combination thereof. In some cases, the transceiver(s) 916 includes other wireless modems, such as a modem for engaging in WI-FI^{®}, WIGIG^{®}, WIMAX^{®}, BLUETOOTH^{®}, or infrared communication over the communication network(s) 920. The signals, in various cases, encode data in the form of data packets, datagrams, or the like. In some cases, the signals are transmitted as compressions are being administered by the chest compression device 900 (e.g., for real-time feedback by the external device(s) 918), after compressions are administered by the chest compression device 900 (e.g., for post-event review at the external device 918), or a combination thereof.

In various cases, the processor(s) 914 generates the control signal based on data encoded in the signals received from the external device(s) 918. For instance, the signals include an instruction to initiate the compressions, and the processor(s) 914 instructs the motor 904 to begin actuating the compressor 902 in accordance with the signals.

In some cases, the chest compression device 900 includes at least one input device 922. In various examples, the input device(s) 922 is configured to receive an input signal from a user 924, who may be a rescuer treating the subject 906. Examples of the input device(s) 922 include, for instance, a switch, a keypad, a cursor control, a touch-sensitive display, a voice input device (e.g., a microphone), a haptic feedback device (e.g., a gyroscope), or any combination thereof. In various implementations, the processor(s) 914 generate the control signal based on the input signal. For instance, the processor(s) 914 generate the control signal to adjust a frequency of the compressions based on the chest compression device 900 detecting a selection by the user 924 of a user interface element displayed on a touchscreen or detecting the user 924 pressing a button integrated with an external housing of the chest compression device 900.

According to some examples, the input device(s) 922 include one or more sensors. The sensor(s), for example, is configured to detect a physiological parameter of the subject 906. In some implementations, the sensor(s) is configured to detect a state parameter of the chest compression device 900, such as a position of the compressor 902 with respect to the subject 906 or the backplate 912, a force administered by the compressor 902 on the subject 906, a force administered onto the backplate 912 by the body of the subject 906 during a compression, or the like. According to some implementations, the signals transmitted by the transceiver(s) 916 indicate the physiological parameter(s) and/or the state parameter(s).

The chest compression device 900 further includes at least one output device 925, in various implementations. Examples of the output device(s) 925 include, for instance, least one of a display (e.g., a projector, an LED screen, etc.), a speaker, a haptic output device, a printer, a light source such as an LED, or any combination thereof. In some implementations, the output device(s) 925 include a screen configured to display various parameters detected by and/or reported to the chest compression device 900, a charge level of the power source 908, a timer indicating a time since compressions were initiated or paused, and other relevant information.

The chest compression device 900 further includes memory 926. In various implementations, the memory 926 is volatile (such as RAM), non-volatile (such as ROM, flash memory, etc.) or some combination of the two. The memory 926 stores instructions that, when executed by the processor(s) 914, causes the processor(s) 914 to perform various operations. In various examples, the memory 926 stores methods, threads, processes, applications, objects, modules, any other sort of executable instruction, or a combination thereof. In some cases, the memory 926 stores files, databases, or a combination thereof. In some examples, the memory 926 includes, but is not limited to, RAM, ROM, 9PROM, flash memory, or any other memory technology. In some examples, the memory 926 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 926 stores instructions, programs, threads, objects, data, or any combination thereof, that cause the processor(s) 914 to perform various functions. In various cases, the memory 926 stores one or more parameters that are detected by the chest compression device 900 and/or reported to the chest compression device 900.

In implementations of the present disclosure, the memory 926 also stores one or more components 928. The component(s) 928 include programs, instructions, files, databases, models, or any other type of data that causes the device 900 to perform any of the functions described herein. For instance, the component(s) 928 may include instructions for performing at least one of the processes 500, 600, or 700.

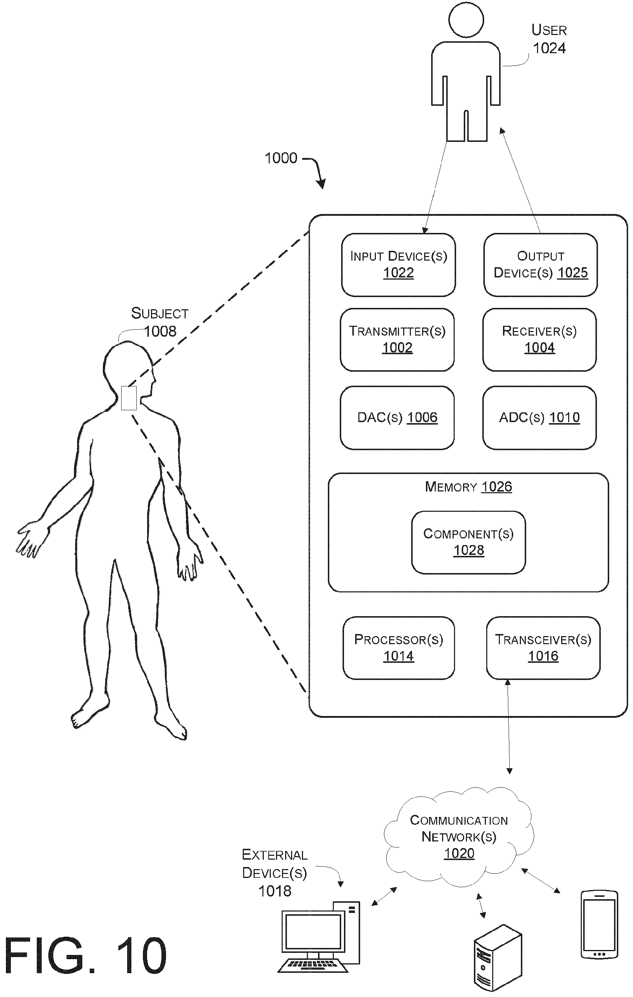

FIG. 10 illustrates a flow monitor 1000 configured to perform various functions described herein. For example, the flow monitor 1000 includes at least one of the first sensor 114, the second sensor 116, the flow monitor 202, the flow monitor 302, or the flow monitor 400.

In various implementations, the flow monitor 1000 includes one or more transmitters 1002, one or more receivers 1004, one or more DACs 1006, and one or more digital to analog converters 1010. The transmitter(s) 1002 is configured to emit an incident beam into a subject 1008. The incident beam includes infrared light and/or ultrasound. For instance, the transmitter(s) 1002 include one or more light sources (e.g., one or more LEDs) or one or more piezoelectric crystals, MEMS transducers, or other electro-mechanical conversion device or material. In some examples, the flow monitor 1000 is positioned such that the incident beam is transmitted through a window in the skull of the subject 1008, such as a temple, an orbital cavity, a nasal cavity, or an ear canal. In some cases, the transmitter(s) 1002 emits one or more incident beams toward an artery supplying blood to a portion of the body of the subject 1008 and a vein transporting blood out of the portion of the body of the subject 1008.

In various implementations, the incident beam is reflected or otherwise scattered by blood in a blood vessel of the subject 1008. The receiver(s) 1004 is configured to detect within a return beam that includes signals from the reflection or scatter from the blood in the blood vessel. For example, the receiver(s) 1004 include one or more light sensors or one or more piezoelectric crystals, MEMS transducers, or other electro-mechanical conversion device or material. In various cases, the flow monitor 1000 includes one or more transceivers that embody the transmitter(s) 1002 and receiver(s) 1004.

The operation of the flow monitor 1000 may be controlled by at least one processor 1014. The processor(s) 1014 are communicatively coupled to the DAC(s) 1006 and the ADC(s) 1010. The DAC(s) 1006 is configured to convert digital signals output by the processor(s) 1014 into analog signals, such as analog signals that induce the transmitter(s) 1002 to emit the incident beam. The ADC(S) 1010 is configured to convert analog signals generated by the receiver(s) 1004 (e.g., induced by detecting the return beam) into digital signals that are received by the processor(s) 1014. The processor(s) 1014 is configured to control the transmitter(s) 1002 and to analyze the signals detected by the receiver(s) 1004. The processor(s) 1014, for instance, analyze the digital signals from the ADC(s) 1010, which are indicative of the return beam detected by the receiver(s) 1004, in order to determine a flow velocity of the blood through the blood vessel. In some examples, the flow monitor 1000 includes one or more switches (e.g., MOSFETs) connected to an array of transmitters within the transmitter(s) 1002 that respectively connect the transmitter(s) 1002 between one or more power rails (e.g., negative and/or positive power rails) and ground. The processor(s) 1014, for instance, cause the switch(es) to connect each transmitter among the transmitter(s) 1002 between ground and the power rail(s) in a periodic waveform, which causes the transmitter(s) 1002 to selectively output incident beam(s) in accordance with the periodic waveform. In some cases, the waveform has a three half-cycle waveform and goes from a positive voltage, to a negative voltage, to a positive voltage, then to ground. In some cases, more than one positive power rail or more than one negative power rail is used for the same waveform.

In various implementations, the flow monitor 1000 includes at least one transceiver 1016 configured to communicate with at least one external device 1018 over one or more communication networks 1020. Any communication network described herein can be included in the communication network(s) 1020 illustrated in FIG. 10. The external device(s) 1018, for example, includes at least one of a mechanical chest compression device, a monitor-defibrillator, an AED, an ECMO device, a ventilation device, a subject monitor, a mobile phone, a server, or a computing device. In some implementations, the transceiver(s) 1016 is configured to communicate with the external device(s) 1018 by transmitting and/or receiving signals in a wired fashion and/or wirelessly. For example, the transceiver(s) 1016 includes a NIC, a network adapter, a LAN adapter, or a physical, virtual, or logical address to connect to the various external devices and/or systems. In various examples, the transceiver(s) 1016 includes any sort of wireless transceivers capable of engaging in wireless communication (e.g., RF communication). For example, the communication network(s) 1020 includes one or more wireless networks that include a 3GPP network, such as an LTE RAN (e.g., over one or more LTE bands), an NR RAN (e.g., over one or more NR bands), or a combination thereof. In some cases, the transceiver(s) 1016 includes other wireless modems, such as a modem for engaging in WI-FI^{®}, WIGIG^{®}, WIMAX^{®}, BLUETOOTH^{®}, or infrared communication over the communication network(s) 1020. The signals, in various cases, encode data in the form of data packets, datagrams, or the like. In some cases, the signals are transmitted as compressions are being administered by the flow monitor 1000 (e.g., for real-time feedback by the external device(s) 1018), after compressions are administered by the flow monitor 1000 (e.g., for post-event review at the external device 1018), or a combination thereof.

In various cases, the processor(s) 1014 generates the control signal based on data encoded in the signals received from the external device(s) 1018. For instance, the signals include an instruction to initiate monitoring, and the processor(s) 1014 outputs a control signal that causes the transmitter(s) 1002 to emit the incident beam.

In some cases, the flow monitor 1000 includes at least one input device 1022. In various examples, the input device(s) 1022 is configured to receive an input signal from a user 1024, who may be a rescuer treating the subject 1008. Examples of the input device(s) 1022 include, for instance, a a keypad, a cursor control, a touch-sensitive display, a voice input device (e.g., a microphone), a haptic feedback device (e.g., a gyroscope), or any combination thereof. In various implementations, the processor(s) 1014 generate the control signal based on the input signal. For instance, the processor(s) 1014 generate the control signal to adjust a frequency of the compressions based on the flow monitor 1000 detecting a selection by the user 1024 of a user interface element displayed on a touchscreen or detecting the user 1024 pressing a button integrated with an external housing of the flow monitor 1000.

According to some examples, the input device(s) 1022 include one or more sensors. The sensor(s), for example, is configured to detect a physiological parameter of the subject 1008. In some cases, the sensor(s) include one or more microphones, one or more accelerometers, one or more oximetry sensors, or one or more ECG sensors. The sensor(s), for instance, is configured to detect one or more physiological parameters. In some implementations, the sensor(s) is configured to detect a state parameter of the flow monitor 1000, such as a position of the flow monitor 1000 with respect to the subject 1008 or the like. According to some implementations, the signals transmitted by the transceiver(s) 1016 indicate the physiological parameter(s) and/or the state parameter(s).

The flow monitor 1000 further includes at least one output device 1025, in various implementations. Examples of the output device(s) 1025 include, for instance, least one of a display (e.g., a projector, an LED screen, etc.), a speaker, a haptic output device, a printer, a light such as an LED, or any combination thereof. In some implementations, the output device(s) 1025 include a screen configured to display various parameters detected by and/or reported to the flow monitor 1000, a battery level of the flow monitor 1000, and other relevant information.

The flow monitor 1000 further includes memory 1026. In various implementations, the memory 1026 is volatile (such as RAM), non-volatile (such as ROM, flash memory, etc.) or some combination of the two. The memory 1026 stores instructions that, when executed by the processor(s) 1014, causes the processor(s) 1014 to perform various operations. In various examples, the memory 1026 stores methods, threads, processes, applications, objects, modules, any other sort of executable instruction, or a combination thereof. In some cases, the memory 1026 stores files, databases, or a combination thereof. In some examples, the memory 1026 includes, but is not limited to, RAM, ROM, 9PROM, flash memory, or any other memory technology. In some examples, the memory 1026 includes one or more of CD-ROMs, DVDs, CAM, or other optical storage, magnetic cassettes, magnetic tape, magnetic disk storage or other magnetic storage devices, or any other medium which can be used to store the desired information. In various cases, the memory 1026 stores instructions, programs, threads, objects, data, or any combination thereof, that cause the processor(s) 1014 to perform various functions. In various cases, the memory 1026 stores one or more parameters that are detected by the flow monitor 1000 and/or reported to the flow monitor 1000.

In implementations of the present disclosure, the memory 1026 also stores one or more components 1028. The component(s) 1028 include programs, instructions, files, databases, models, or any other type of data that causes the flow monitor 1000 to perform any of the functions described herein. For instance, the component(s) 1028 may include instructions for performing at least one of the processes 500, 600, or 700.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of ±20% of the stated value; ±19% of the stated value; ±18% of the stated value; ±17% of the stated value; ±16% of the stated value; ±15% of the stated value; ±14% of the stated value; ±13% of the stated value; ±12% of the stated value; ±11% of the stated value; ±10% of the stated value; ±9% of the stated value; ±8% of the stated value; ±7% of the stated value; ±6% of the stated value; ±5% of the stated value; ±4% of the stated value; ±3% of the stated value; ±2% of the stated value; or ±1% of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

## Claims

1. A medical device (106, 202, 228, 800), comprising:
a first sensor (114) configured to detect, during a time interval, a sound indicative of cardiac activity of a subject;
a second sensor (116) configured to detect, during the time interval, an additional physiological parameter of the subject;
a processor configured to generate audio (118) indicative of a pulse of the subject by analyzing the sound indicative of cardiac activity of the subject;
a speaker (224) configured to output the audio indicative of the pulse of the subject; and
a display (112) configured to output a visual signal indicating the additional physiological parameter of the subject detected during the time interval, the display (112) outputting the visual signal simultaneously as the speaker (224) is outputting the audio indicative of the pulse of the subject, **characterized in that** the processor is configured to generate the audio indicative of the pulse of the subject by analyzing the sound indicative of cardiac activity of the subject by:
determining a difference between a rate of the pulse and a reference value; and
generating the audio to have a pitch that is proportional to the difference.

2. The medical device (106, 202, 228, 800) of claim 1, wherein the sound indicative of cardiac activity of the subject comprises a reflection of an incident beam (114, 216) from a heart of the subject.

3. The medical device (106, 202, 228, 800) of claim 2, wherein the first sensor (114) comprises an ultrasound transducer and the incident beam (114, 216) comprises ultrasound.

4. The medical device (106, 202, 228, 800) of claim 2 or 3, wherein the processor is configured to generate the audio indicative of the pulse of the subject by:
determining a velocity of a heart wall of the subject by comparing a frequency of the reflection of the incident beam (114, 216) to a frequency of the incident beam; and
defining a pitch or volume of the audio to be proportional to the velocity of the heart wall of the subject.

5. The medical device (106, 202, 228, 800) of claim 2, 3 or 4, wherein the reflection of the incident beam (114, 216) is from:
blood flowing through a blood vessel of the subject;
blood flowing through a heart of the subject; and/or
a wall of the heart of the subject.

6. The medical device (106, 202, 228, 800) of any of claims 1-5, wherein the additional physiological parameter comprises an electrocardiogram (ECG), a blood oxygenation, or a capnograph.

7. The medical device (106, 202, 228, 800) of any of claims 1-6, wherein the audio indicative of the pulse of the subject is further indicative of an arrhythmia of a heart of the subject during the time interval.

8. The medical device (106, 202, 228, 800) of any of claims 1-7, further comprising:
a monitoring circuit configured to detect an electrocardiogram (ECG) of a subject; and
an input device (818) configured to receive an input signal from a user;
wherein the display (820) is configured to visually output the ECG and the physiological parameter;
wherein the processor (812) is configured to determine that the ECG is indicative of an arrhythmia; and further comprising: a treatment circuit (825) configured to, in response to the processor determining that the ECG is indicative of an arrhythmia and the input device receiving the input signal from the user, output an electrical shock to the heart of the subject.

9. The medical device (106, 202, 228, 800) of claim 8, wherein the first sensor comprises:
a transmitter (212) configured to output ultrasound toward the heart of the subject;
a receiver (222) configured to detect a reflection of the ultrasound from blood in the heart of the subject, the sound being the reflection of the ultrasound from the blood in the heart of the subject;
a housing; and
an adhesive (206) disposed on the housing, the adhesive attaching the first sensor to skin on a chest of the subject, and
wherein the processor is further configured to:
determine a velocity of the blood in the heart of the subject over time by analyzing the sound; and
converting the velocity of the blood in the heart of the subject over time into the audio.

10. A method performed by a single medical device (106, 202, 228, 800), the method comprising:
receiving, during a time interval, a sound indicative of cardiac activity of a subject;
generating audio indicative of a pulse of the subject by analyzing the sound; and
simultaneously outputting:
the audio indicative of the pulse of the subject; and
a visual signal indicating an additional physiological parameter of the subject received during the time interval, **characterized in that** generating the audio indicative of the pulse of the subject by analyzing the sound indicative of cardiac activity of the subject comprises:
determining a difference between a rate of the pulse and a reference value; and
defining a pitch or volume of the audio to be proportional to the difference.

11. The method of claim 10, wherein the sound indicative of cardiac activity of the subject comprises a reflection of an incident beam from a heart of the subject;
wherein optionally a) the incident beam comprises ultrasound; and/or
b) generating the audio indicative of the pulse of the subject comprises:
determining a velocity of a wall of a heart of the subject by comparing a frequency of the reflection of the incident beam to a frequency of the incident beam; and
defining a pitch or volume of the audio to be proportional to the velocity of the wall of the heart of the subject; and/or
c) the reflection of the incident beam is from blood flowing through a heart of the subject or a blood vessel of the subject; and/or
wherein the sound indicative of cardiac activity of the subject is generated by movement of a heart of the subject and/or by blood flowing through a blood vessel of the subject.

12. The method of any of claims 10-11, wherein the additional physiological parameter comprises an electrocardiogram (ECG), a blood oxygenation, or a capnograph.

13. The method of any of claims 10-12, wherein the audio indicative of the pulse of the subject is further indicative of an arrhythmia of a heart of the subject during the time interval.

## Patentansprüche

1. Medizinisches Gerät (106, 202, 228, 800), umfassend:
einen ersten Sensor (114), der dazu konfiguriert ist, während eines Zeitintervalls ein Geräusch zu erfassen, das indikativ für die Herzaktivität eines Subjekts ist;
einen zweiten Sensor (116), der dazu konfiguriert ist, während des Zeitintervalls einen zusätzlichen physiologischen Parameter des Subjekts zu erfassen;
einen Prozessor, der dazu konfiguriert ist, einen Ton (118) zu erzeugen, der indikativ für den Puls des Subjekts ist, durch Analysieren des Geräusches, das indikativ für die Herzaktivität des Subjekts ist;
einen Lautsprecher (224), der dazu konfiguriert ist, den Ton auszugeben, der indikativ für den Puls des Subjekts ist; und
eine Anzeige (112), die dazu konfiguriert ist, ein visuelles Signal auszugeben, das den während des Zeitintervalls erfassten zusätzlichen physiologischen Parameter des Subjekts angibt, wobei die Anzeige (112) das visuelle Signal gleichzeitig mit dem von dem Lautsprecher (224) ausgegebenen Ton ausgibt, der indikativ für den Puls des Subjekts ist, **dadurch gekennzeichnet, dass** der Prozessor dazu konfiguriert ist, den Ton zu erzeugen, der indikativ für den Puls des Subjekts ist, durch Analysieren des Geräusches, das indikativ für die Herzaktivität des Subjekts ist, durch:
Bestimmen einer Differenz zwischen einer Pulsfrequenz und einem Referenzwert; und
Erzeugen des Tons, sodass er eine Tonhöhe aufweist, die proportional zu der Differenz ist.

2. Medizinisches Gerät (106, 202, 228, 800) nach Anspruch 1, wobei das Geräusch, das indikativ für die Herzaktivität des Subjekts ist, eine Reflexion eines einfallenden Strahls (114, 216) von einem Herzen des Subjekts umfasst.

3. Medizinisches Gerät (106, 202, 228, 800) nach Anspruch 2, wobei der erste Sensor (114) einen Ultraschallwandler aufweist und der einfallende Strahl (114, 216) Ultraschall umfasst.

4. Medizinisches Gerät (106, 202, 228, 800) nach Anspruch 2 oder 3, wobei der Prozessor dazu konfiguriert ist, den Ton zu erzeugen, der indikativ für den Puls des Subjekts ist, durch:
Bestimmen einer Geschwindigkeit einer Wand eines Herzens des Subjekts durch Vergleichen einer Frequenz der Reflexion des einfallenden Strahls (114, 216) mit einer Frequenz des einfallenden Strahls; und
Definieren einer Tonhöhe oder Lautstärke des Tons, um proportional zu der Geschwindigkeit der Wand eines Herzens des Subjekts zu sein.

5. Medizinisches Gerät (106, 202, 228, 800) nach Anspruch 2, 3 oder 4, wobei die Reflexion des einfallenden Strahls (114, 216) von Folgendem stammt:
Blut, das durch ein Blutgefäß des Subjekts fließt;
Blut, das durch das Herz des Subjekts fließt; und/oder
eine Wand des Herzens des Subjekts.

6. Medizinisches Gerät (106, 202, 228, 800) nach einem der Ansprüche 1 bis 5, wobei der zusätzliche physiologische Parameter ein Elektrokardiogramm (EKG), eine Blutsauerstoffsättigung oder ein Kapnogramm umfasst.

7. Medizinisches Gerät (106, 202, 228, 800) nach einem der Ansprüche 1 bis 6, wobei der Ton, der indikativ für den Puls des Subjekts ist, ferner indikativ für eine Arrhythmie eines Herzens des Subjekts während des Zeitintervalls ist.

8. Medizinisches Gerät (106, 202, 228, 800) nach einem der Ansprüche 1 bis 7, ferner umfassend:
eine Überwachungsschaltung, die dazu konfiguriert ist, ein Elektrokardiogramm (EKG) eines Subjekts zu erfassen; und
ein Eingabegerät (818), das dazu konfiguriert ist, ein Eingangssignal von einem Benutzer zu empfangen;
wobei der Prozessor (812) dazu konfiguriert ist, zu bestimmen, dass das EKG indikativ für eine Arrhythmie ist; und ferner umfassend:
eine Behandlungsschaltung (825), die dazu konfiguriert ist, als Reaktion auf das Bestimmen des Prozessors, dass das EKG indikativ für eine Arrhythmie ist, und das Empfangen des Eingangssignals von dem Benutzer durch das Eingabegerät, einen elektrischen Schock an das Herz des Subjekts auszugeben.

9. Medizinisches Gerät (106, 202, 228, 800) nach Anspruch 8, wobei der erste Sensor Folgendes aufweist:
einen Sender (212), der dazu konfiguriert ist, Ultraschall in Richtung des Herzens des Subjekts auszugeben;
einen Empfänger (222), der dazu konfiguriert ist, eine Reflexion des Ultraschalls von Blut in dem Herzen des Subjekts zu erfassen, wobei das Geräusch die Reflexion des Ultraschalls von Blut in dem Herzen des Subjekts ist;
ein Gehäuse; und
einen Klebstoff (206), der auf dem Gehäuse angeordnet ist, wobei der Klebstoff den ersten Sensor an der Haut auf einer Brust des Subjekts befestigt, und
wobei der Prozessor ferner konfiguriert ist, zum:
Bestimmen einer Geschwindigkeit des Blutes in dem Herzen des Subjekts über einen Zeitraum hinweg durch Analysieren des Geräusches; und
Umwandeln der Geschwindigkeit des Blutes in dem Herzen des Subjekts über einen Zeitraum hinweg in den Ton.

10. Verfahren, das von einem einzigen medizinischen Gerät (106, 202, 228, 800) durchgeführt wird, wobei das Verfahren Folgendes umfasst:
Empfangen, während eines Zeitintervalls, eines Geräusches, das indikativ für Herzaktivität eines Subjekts ist;
Erzeugen von Ton, der indikativ für einen Puls des Subjekts ist, durch Analysieren des Geräusches, und
gleichzeitiges Ausgeben:
des Tons, der indikativ für den Puls des Subjekts ist; und eines visuellen Signals, das einen während des Zeitintervalls empfangenen zusätzlichen physiologischen Parameter des Subjekts angibt, **dadurch gekennzeichnet, dass** das Erzeugen des Tons, der indikativ für den Puls des Subjekts ist, durch Analysieren des Geräusches, das indikativ für die Herzaktivität des Subjekts ist, Folgendes umfasst:
Bestimmen einer Differenz zwischen einer Pulsfrequenz und einem Referenzwert; und
Definieren einer Tonhöhe oder Lautstärke des Tons, um proportional zu der Differenz zu sein.

11. Verfahren nach Anspruch 10, wobei das Geräusch, das indikativ für Herzaktivität des Subjekts ist, eine Reflexion eines einfallenden Strahls von einem Herzen des Subjekts umfasst;
wobei optional a) der einfallende Strahl Ultraschall umfasst; und/oder
b) das Erzeugen des Tons, der indikativ für den Puls des Subjekts ist, Folgendes umfasst:
Bestimmen einer Geschwindigkeit einer Wand eines Herzens des Subjekts durch Vergleichen einer Frequenz der Reflexion des einfallenden Strahls mit einer Frequenz des einfallenden Strahls; und
Definieren einer Tonhöhe oder Lautstärke des Tons, um proportional zu der Geschwindigkeit der Wand des Herzens des Subjekts zu sein; und/oder
c) die Reflexion des einfallenden Strahls von Blut stammt, das durch ein Herz des Subjekts oder ein Blutgefäß des Subjekts fließt; und/oder
wobei das Geräusch, das indikativ für Herzaktivität des Subjekts ist, durch Bewegung eines Herzens des Subjekts und/oder durch Blut, das durch ein Blutgefäß des Subjekts fließt, erzeugt wird.

12. Verfahren nach einem der Ansprüche 10 bis 11, wobei der zusätzliche physiologische Parameter ein Elektrokardiogramm (EKG), eine Blutsauerstoffsättigung oder ein Kapnogramm umfasst.

13. Verfahren nach einem der Ansprüche 10 bis 12, wobei der Ton, der indikativ für den Puls des Subjekts ist, ferner indikativ für eine Arrhythmie eines Herzens des Subjekts während des Zeitintervalls ist.

## Revendications

1. Dispositif médical (106, 202, 228, 800) comprenant :
un premier capteur (114) configuré pour détecter, pendant un intervalle de temps, un son indicatif d'une activité cardiaque d'un sujet ;
un deuxième capteur (116) configuré pour détecter, pendant l'intervalle de temps, un paramètre physiologique supplémentaire du sujet ;
un processeur configuré pour générer un audio (118) indicatif d'un pouls du sujet en analysant le son indicatif d'une activité cardiaque du sujet ;
un haut-parleur (224) configuré pour émettre l'audio indicatif du pouls du sujet ; et
un affichage (112) configuré pour émettre un signal visuel indiquant le paramètre physiologique supplémentaire du sujet détecté pendant l'intervalle de temps, l'affichage (112) émettant le signal visuel simultanément à l'émission par le haut-parleur (224) de l'audio indicatif du pouls du sujet, **caractérisé en ce que** le processeur est configuré pour générer l'audio indicatif du pouls du sujet en analysant le son indicatif d'une activité cardiaque du sujet :
en déterminant une différence entre un taux du pouls et une valeur de référence ; et
en générant l'audio pour avoir une hauteur qui est proportionnelle à la différence.

2. Dispositif médical (106, 202, 228, 800) selon la revendication 1, dans lequel le son indicatif d'une activité cardiaque du sujet comprend une réflexion d'un faisceau incident (114, 216) provenant du cœur du sujet.

3. Dispositif médical (106, 202, 228, 800) selon la revendication 2, dans lequel le premier capteur (114) comprend un transducteur à ultrasons et le faisceau incident (114, 216) comprend des ultrasons.

4. Dispositif médical (106, 202, 228, 800) selon la revendication 2 ou 3, dans lequel le processeur est configuré pour générer l'audio indicatif du pouls du sujet :
en déterminant une vitesse d'une paroi cardiaque du sujet en comparant une fréquence de la réflexion du faisceau incident (114, 216) à une fréquence du faisceau incident ; et
en définissant une hauteur ou un volume de l'audio de façon à ce qu'elle ou il soit proportionnel(le) à la vitesse de la paroi cardiaque du sujet.

5. Dispositif médical (106, 202, 228, 800) selon la revendication 2, 3 ou 4, dans lequel la réflexion du faisceau incident (114, 216) provient :
du sang circulant dans un vaisseau sanguin du sujet ;
du sang circulant dans le cœur du sujet ; et/ou
d'une paroi cardiaque du sujet.

6. Dispositif médical (106, 202, 228, 800) selon l'une des revendications 1 à 5, dans lequel le paramètre physiologique supplémentaire comprend un électrocardiogramme (ECG), une oxygénation du sang ou une capnographie.

7. Dispositif médical (106, 202, 228, 800) selon l'une des revendications 1 à 6, dans lequel l'audio indicatif du pouls du sujet est en outre indicatif d'une arythmie du cœur du sujet pendant l'intervalle de temps.

8. Dispositif médical (106, 202, 228, 800) selon l'une des revendications 1 à 7, comprenant en outre :
un circuit de surveillance configuré pour détecter un électrocardiogramme (ECG) d'un sujet ; et
un dispositif d'entrée (818) configuré pour recevoir un signal d'entrée d'un utilisateur ;
dans lequel l'affichage (820) est configuré pour délivrer visuellement l'ECG et le paramètre physiologique ;
dans lequel le processeur (812) est configuré pour déterminer que l'ECG est indicatif d'une arythmie ; et comprenant en outre :
un circuit de traitement (825) configuré pour, lorsque le processeur détermine que l'ECG est indicatif d'une arythmie et que le dispositif d'entrée reçoit le signal d'entrée de l'utilisateur, délivrer un choc électrique au cœur du sujet.

9. Dispositif médical (106, 202, 228, 800) selon la revendication 8, dans lequel le premier capteur comprend :
un émetteur (212) configuré pour émettre des ultrasons vers le cœur du sujet ;
un récepteur (222) configuré pour détecter une réflexion des ultrasons provenant du sang dans le cœur du sujet, le son étant la réflexion des ultrasons provenant du sang dans le cœur du sujet ;
un boîtier ; et
un adhésif (206) disposé sur le boîtier, l'adhésif fixant le premier capteur à la peau sur la poitrine du sujet, et
dans lequel le processeur est en outre configuré pour :
déterminer une vitesse du sang dans le cœur du sujet au fil du temps en analysant le son ; et
convertir la vitesse du sang dans le cœur du sujet au fil du temps dans l'audio.

10. Procédé réalisé par un seul dispositif médical (106, 202, 228, 800), le procédé comprenant les étapes suivantes :
recevoir, pendant un intervalle de temps, un son indicatif d'une activité cardiaque d'un sujet ;
générer un audio indicatif d'un pouls du sujet en analysant le son ; et
émettre simultanément :
l'audio indicatif du pouls du sujet ; et
un signal visuel indiquant un paramètre physiologique supplémentaire du sujet reçu pendant l'intervalle de temps, **caractérisé en ce que**
la génération de l'audio indicatif du pouls du sujet en analysant le son indicatif d'une activité cardiaque du sujet comprend :
la détermination d'une différence entre un taux du pouls et une valeur de référence ; et
la définition d'une hauteur ou d'un volume de l'audio de façon à ce qu'elle ou il soit proportionnel(le) à la différence.

11. Procédé selon la revendication 10, dans lequel le son indicatif d'une activité cardiaque du sujet comprend une réflexion d'un faisceau incident provenant du cœur du sujet ;
dans lequel facultativement
a) le faisceau incident comprend des ultrasons ; et/ou
b) la génération de l'audio indicatif du pouls du sujet comprend :
la détermination d'une vitesse d'une paroi cardiaque du sujet en comparant une fréquence de la réflexion du faisceau incident à une fréquence du faisceau incident ; et
la définition d'une hauteur ou d'un volume de l'audio de façon à ce qu'elle ou il soit proportionnel(le) à la vitesse de la paroi cardiaque du sujet ; et/ou
c) la réflexion du faisceau incident provient du sang circulant dans un cœur du sujet ou d'un vaisseau sanguin du sujet ; et/ou
dans lequel le son indicatif d'une activité cardiaque du sujet est généré par le mouvement du cœur du sujet et/ou par le sang circulant dans un vaisseau sanguin du sujet.

12. Procédé selon l'une des revendications 10 et 11, dans lequel le paramètre physiologique supplémentaire comprend un électrocardiogramme (ECG), une oxygénation du sang ou une capnographie.

13. Procédé selon l'une des revendications 10 à 12, dans lequel l'audio indicatif du pouls du sujet est en outre indicatif d'une arythmie du cœur du sujet pendant l'intervalle de temps.
